(19) Europäisches Patentamt
European Patent Office
Office européen des brevets

(11) Publication number: **0 105 732 A2**

(12) **EUROPEAN PATENT APPLICATION**

(21) Application number: 83305899.3

(22) Date of filing: 29.09.83

(51) Int. Cl.[3]: C 07 D 417/12
C 07 D 275/06, C 07 D 285/32
A 61 K 31/425, A 61 K 31/445
A 61 K 31/54

(30) Priority: 01.10.82 GB 8228150
19.02.83 GB 8304645
28.07.83 GB 8320326

(43) Date of publication of application: 18.04.84 Bulletin 84/16

(84) Designated Contracting States: CH DE FR GB IT LI NL

(71) Applicant: BEECHAM GROUP PLC
Beecham House Great West Road
Brentford Middlesex(GB)

(72) Inventor: King, Francis David
67 Cherry Garden Lane
Newport Essex(GB)

(72) Inventor: Fake, Charles Sylvester
5 Mayfield Close
Harlow Essex(GB)

(72) Inventor: Burrell, Gordon
11 Mercers
Harlow Essex(GB)

(74) Representative: Stott, Michael John et al,
European Patent Attorney Beecham Pharmaceuticals
Great Burgh Yew Tree Bottom Road
Epsom Surrey KT18 5XQ(GB)

(54) **Histamine H2 receptor antagonists of the benzisothiazole amino and benzthiadiazine amino series.**

(57) Compounds of formula (I), or a pharmaceutically acceptable salt, quaternised derivative, N-oxide or solvate thereof:

$R_1$–Ar–$(CH_2)_a$–X–$(CH_2)_b$–NH–Het (I)

wherein:

$R_1$ is hydrogen, halogen, hydroxy, $C_{1-4}$ alkyl, $C_{1-4}$ alkoxy, or amino or guanidino, the or any amino moiety being optionally substituted by one or two $C_{1-4}$ alkyl optionally substituted by halogen, or an aminomethylene group of formula (a),

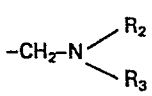

(a)

in which $R_2$ and $R_3$ are the same or different and are hydrogen, $C_{1-4}$ alkyl, $C_{3-6}$ cycloalkyl, $C_{3-6}$ cycloalkyl-$C_{1-4}$ alkyl or phenyl $C_{1-4}$ alkyl, or $R_2$ and $R_3$ together with the nitrogen atom to which they are attached are morpholino, pyrrolidino or piperidino;

Ar is furandiyl, thiophendiyl, phenylene, pyridinediyl, pyrimidinediyl, thiazolediyl, thiadiazolediyl or imidazolediyl;

a is 0 or an integer 1 or 2;

X is –S–, –O–, –$CH_2$–, $-\overset{O}{\overset{\|}{S}}-$ or –NH–;

b is an integer from 2 to 5; and
Het is a bicyclic heteroaryl group of formula (b) or (c),

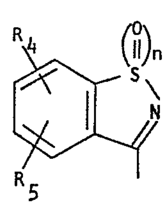

(b)

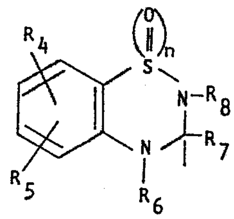

(c)

./...

EP 0 105 732 A2

in which $R_4$ and $R_5$ are the same or different and are hydrogen, halogen, $C_{1-6}$ alkyl, trifluoromethyl, nitro, cyano, hydroxy, $C_{1-6}$ alkoxy, $C_{1-6}$ alkylthio, carboxy, amino, aminocarbonyl, aminocarbonylamino, amino-thiocarbonylamino, aminosulphonyl, $C_{1-6}$ alkylsulphonyl, $C_{1-6}$ alkylsulphinyl, $C_{1-6}$ alkylsulphonylamino, $C_{1-6}$ alkyluslphinylamino, phenylsulphonyl, phenylsulphinyl or are a group of formula (d) or (e),

$R_9$–CY– (d) $R_9$–CY–Z– (e)

in which $R_9$ is hydrogen, $C_{1-6}$ alkyl, $C_{1-6}$ alkoxy, $C_{1-6}$ alkylthio, phenyl, phenoxy or phenylthio, Y is oxygen or sulphur and Z is oxygen, sulphur or N-$R_{10}$, $R_{10}$ being hydrogen or $C_{1-6}$ alkyl, the $C_{1-6}$ alkyl group or the $C_{1-6}$ alkyl moiety of any of the foregoing $C_{1-6}$ alkyl-containing groups for $R_4$ and $R_5$ being optionally substituted by phenyl, the amino group or the amino moiety of any of the foregoing amino-containing groups for $R_4$ and $R_5$ being optionally substituted by one or two $C_{1-6}$ alkyl, phenyl or phenyl-$C_{1-4}$ alkyl or optionally N-disubstituted by $C_{4-5}$ polymethylene, and the phenyl moity of any of the foregoing phenyl-containing groups for $R_4$ and $R_5$ being optionally substituted by $C_{1-6}$ alkyl, $C_{1-6}$ alkoxy, halogen, nitro or trifluoromethyl, one of $R_6$ and $R_8$ is hydrogen, $C_{1-6}$ alkyl, $C_{1-6}$ alkanoyl, $C_{1-6}$ alkylsulphonyl, $C_{1-6}$ alkoxycarbonyl or phenoxycarbonyl, benzyloxycarbonyl, phenyl, phenylsulphonyl or phenyl $C_{1-4}$ alkyl, the phenyl moiety being optionally substituted by benzoyl, $C_{1-6}$ alkyl, $C_{1-4}$ alkoxy, halogen, nitro or trifluoromethyl, and the other of $R_6$ and $R_8$ together with $R_7$ is a bond, and n is O or an integer 1 or 2.

TITLE MODIFIED
see front page

## NOVEL COMPOUNDS

The present invention relates to novel compounds having pharmacological activity, to processes and intermediates for their preparation, to pharmaceutical compositions containing them, and to their use in the treatment of mammals.

U.S. Patent 4 128 658 discloses a class of aminoalkylfuran derivatives that are histamine $H_2$-receptor antagonists and that, therefore, are useful in the treatment of disorders relating to excess gastric acid secretion. One such derivative is the compound, N-[2-[[[5-(dimethylamino)methyl-2-furanyl]methyl]thio]ethyl]-N'-methyl-2-nitro-1,1-ethenediamine, which is commonly known as ranitidine.

A structurally distinct class of compounds has now been discovered. In addition, such compounds have been found to be histamine $H_2$-receptor antagonists. They are, therefore, useful in the treatment of disorders relating to excess gastric acid secretion, such as peptic ulcer and Zollinger-Ellison syndrome.

Accordingly, the present invention provides a compound of formula (I), or a pharmaceutically acceptable salt, quaternised derivative, N-oxide or solvate therof:

$$R_1\text{-Ar-}(CH_2)_a\text{-X-}(CH_2)_b\text{-NH-Het} \qquad (I)$$

wherein:

$R_1$ is hydrogen, halogen, hydroxy, $C_{1-4}$ alkyl, $C_{1-4}$ alkoxy, or amino or guanidino, the or any amino moiety being optionally substituted by one or two $C_{1-4}$ alkyl optionally substituted by halogen, or an aminomethylene group of formula (a),

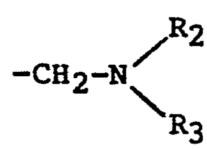

(a)

in which $R_2$ and $R_3$ are the same or different and are hydrogen, $C_{1-4}$ alkyl, $C_{3-6}$ cycloalkyl, $C_{3-6}$ cycloalkyl-$C_{1-4}$ alkyl or phenyl $C_{1-4}$ alkyl, or $R_2$ and $R_3$ together with the nitrogen atom to which they are attached are morpholino, pyrrolidino or piperidino;

Ar is furandiyl, thiophendiyl, phenylene, pyridinediyl, pyrimidinediyl, thiazolediyl, thiadiazolediyl or imidazolediyl;

a is 0 or an integer 1 or 2;

X is -S-, -O-, $-CH_2-$, $-\overset{\overset{O}{\|}}{S}-$ or -NH-;

b is an integer from 2 to 5; and

Het is a bicyclic heteroaryl group of formula (b) or (c),

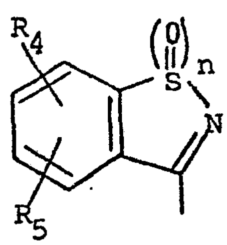

(b)

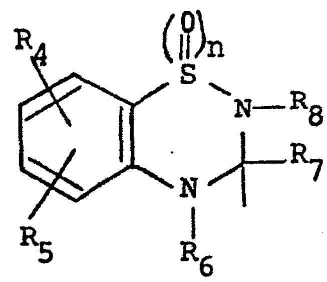

(c)

in which $R_4$ and $R_5$ are the same or different and are hydrogen, halogen, $C_{1-6}$ alkyl, trifluoromethyl, nitro, cyano, hydroxy, $C_{1-6}$ alkoxy, $C_{1-6}$ alkylthio, carboxy, amino, aminocarbonyl, aminocarbonylamino, amino-thiocarbonylamino, aminosulphonyl, $C_{1-6}$ alkylsulphonyl, $C_{1-6}$ alkylsulphinyl, $C_{1-6}$ alkylsulphonylamino, $C_{1-6}$ alkylsulphinylamino, phenylsulphonyl, phenylsulphinyl or are a group of formula (d) or (e),

$R_9$-CY- (d) $R_9$-CY-Z- (e)

in which $R_9$ is hydrogen, $C_{1-6}$ alkyl, $C_{1-6}$ alkoxy,

$C_{1-6}$ alkylthio, phenyl, phenoxy or phenylthio, Y is oxygen or sulphur and Z is oxygen, sulphur or N-$R_{10}$, $R_{10}$ being hydrogen or $C_{1-6}$ alkyl, the $C_{1-6}$ alkyl group or the $C_{1-6}$ alkyl moiety of any of the foregoing $C_{1-6}$ alkyl-containing groups for $R_4$ and $R_5$ being optionally substituted by phenyl, the amino group or the amino moiety of any of the foregoing amino-containing groups for $R_4$ and $R_5$ being optionally substituted by one or two $C_{1-6}$ alkyl, phenyl or phenyl-$C_{1-4}$ alkyl or optionally N-disubstituted by $C_{4-5}$ polymethylene, and the phenyl moiety of any of the foregoing phenyl-containing groups for $R_4$ and $R_5$ being optionally substituted by $C_{1-6}$ alkyl, $C_{1-6}$ alkoxy, halogen, nitro or trifluoromethyl, one of $R_6$ and $R_8$ is hydrogen, $C_{1-6}$ alkyl, $C_{1-6}$ alkanoyl, $C_{1-6}$ alkylsulphonyl, $C_{1-6}$ alkoxycarbonyl or phenoxycarbonyl, benzyloxycarbonyl, phenyl, phenylsulphonyl or phenyl $C_{1-4}$ alkyl, the phenyl moiety being optionally substituted by benzoyl, $C_{1-6}$ alkyl, $C_{1-4}$ alkoxy, halogen, nitro or trifluoromethyl, and the other of $R_6$ and $R_8$ together with $R_7$ is a bond, and n is O or an integer 1 or 2.

Examples of Ar include 2,5-furandiyl; 2,5-thiophendiyl; 1,3- and 1,4-phenylene; 2,3- and 2,4-pyridinediyl; 2,4-pyrimidinediyl; 2,4-thiazolediyl; 3,5-(1,2,4,)-thiadiazolediyl; and 2,4- and 4,5-imidazolediyl. Preferred examples of Ar include 2,5-furandiyl; 2,5-thiophendiyl; 1,3-phenylene; and 2,4-thiazolediyl. The most preferred example of Ar is 2,5-furandiyl.

When Ar is furandiyl, thiophendiyl or phenylene, $R_1$ is, preferably, an aminomethylene group of formula (a), as hereinbefore defined. In particular, $R_1$ is an aminomethylene group of formula (a), in which $R_2$ and $R_3$ are the same or different and are hydrogen, methyl, ethyl, cyclopropyl or cyclohexyl, or $R_2$ and $R_3$ together with the nitrogen atom to which they are attached are pyrrolidino or piperidino. Most preferably, $R_1$ is dimethylaminomethyl, pyrrolidinomethyl or piperidinomethyl.

When Ar is pyridinediyl, pyrimidinediyl, thiazolediyl, thiadiazolediyl or imidazolediyl, in particular thiazolediyl, $R_1$ is, preferably, hydrogen, halogen, hydroxy, $C_{1-4}$ alkyl, $C_{1-4}$ alkoxy, or amino or guanidino, the or any amino moiety being optionally substituted by one or two $C_{1-4}$ alkyl optionally substituted by halogen, or an aminomethylene group of formula (a), as defined hereinbefore. In particular, $R_1$ is hydrogen, halogen, such as bromo, $C_{1-4}$ alkyl, such as methyl, or guanidino, any amino moiety being optionally substituted by one or two $C_{1-4}$ alkyl.

Preferred examples of a are O and the integer 1. In relation to the preferred examples of Ar, a is, preferably, the integer 1 when Ar is 2,5-furandiyl, 2,5-thiophendiyl or 2,4-thiazolediyl and is, preferably, O when Ar is 1,3-phenylene.

Preferred examples of X include -S-, -O- and $-CH_2-$, in particular -S- and -O-.

Preferred examples of b are the integers 2, 3 and 4, especially the integers 2 and 3. In relation to the preferred examples of Ar, b is, preferably, the integer 2 when Ar is 2,5-furandiyl, 2,5-thiophendiyl or 2,4-thiazolediyl, and is, preferably, the integer 3 when Ar is 1,3-phenylene.

A sub-class of $R_4$ and $R_5$ is that wherein $R_4$ and $R_5$ are the same or different and are hydrogen, trifluoromethyl, nitro, cyano, hydroxy, $C_{1-6}$ alkoxy, $C_{1-6}$ alkylthio, or amino optionally substituted by one or two $C_{1-6}$ alkyl or phenyl $C_{1-4}$ alkyl or optionally N-disubstituted by $C_{4-5}$ polymethylene, or $C_{1-6}$ alkylsulphonyl or phenylsulphonyl, the phenyl moiety being optionally substituted by $C_{1-6}$ alkyl, $C_{1-6}$ alkoxy, halogen, nitro or trifluoromethyl, or are a group of formula (d) in which Y is oxygen and $R_9$ is as defined hereinbefore, or are a group of formula (e) in which Y is oxygen, Z is N-$R_{10}$ and $R_9$ and $R_{10}$ are as defined hereinbefore.

A further sub-class of $R_4$ and $R_5$ is that wherein $R_4$ and $R_5$ are the same or different and are hydrogen, halogen, $C_{1-6}$ alkyl, trifluoromethyl nitro, cyano, $C_{1-6}$ alkoxy, or amino, aminocarbonylamino or aminosulphonyl, the amino moiety being optionally substituted by one or two $C_{1-6}$ alkyl, phenyl or phenyl-$C_{1-4}$ alkyl or optionally N-disubstituted by $C_{4-5}$ polymethylene, or $C_{1-6}$ alkylsulphonylamino or a group of formula (e) in which $R_9$ is hydrogen or $C_{1-6}$ alkyl, Y is oxygen and Z is NH.

Particular examples of $R_4$ and $R_5$, which may be the same or different, include hydrogen, chloro, bromo, iodo, methyl, trifluoromethyl, nitro, cyano, methoxy, amino, methylaminocarbonylamino, aminosulphonyl,

methylaminosulphonyl, dimethylaminosulphonyl, methylsulphonylamino, formamido, acetamido and propionamido.

Preferred examples of $R_4$ and $R_5$, which may be the same or different, include hydrogen, nitro, amino, methylaminosulphonyl, dimethylaminosulphonyl, acetamido and propionamido. In particular, one of $R_4$ and $R_5$ is hydrogen and the other is hydrogen, nitro, amino, methylaminosulphonyl, dimethylaminosulphonyl, acetamido and propionamido. When Het is a bicyclic heteroaryl group of formula (b), the preferred position for substitution by the other of $R_4$ and $R_5$ is the 4- or 6-position, especially the 4-position. When Het is a bicyclic heteroaryl group of formula (c), the preferred position for substitution by the other of $R_4$ and $R_5$ is the 7-position.

Preferably, one of $R_6$ and $R_8$ in the bicyclic heteroaryl group of formula (c) is hydrogen or $C_{1-6}$ alkyl, such as methyl, and the other of $R_6$ and $R_8$ together with $R_7$ is a bond. When one of $R_6$ and $R_8$ is $C_{1-6}$ alkyl, such as methyl, it is preferred that $R_6$ is so defined and $R_8$ together with $R_7$ is a bond.

Preferably, n is an integer 1 or 2, in particular 2.

Preferably, Het is a bicyclic heteroaryl group of formula (b).

The compounds of the present invention are capable of existing as tautomers. The present invention extends to all such tautomers individually and as mixtures.

A pharmaceutically acceptable salt of a compound of the present invention is, preferably, a pharmaceutically acceptable acid addition salt. Examples of such a salt include an inorganic acid addition salt, such as a sulphate, nitrate, phosphate, borate, hydrochloride and hydrobromide, and an organic acid addition salt, such as an acetate, fumarate, tartrate, citrate, succinate, benzoate, ascorbate, methylsulphonate, α-ketoglutarate, α-glycerophosphate and glucose-1-phosphate. The hydrochloride salt is the most preferred pharmaceutically acceptable salt.

A quaternised derivative of a compound of the present invention includes a compound of the present invention quaternised by an optionally substituted alkyl halide, for example a $C_{1-4}$ alkyl, $C_{3-6}$ cycloalkyl, $C_{3-6}$ cycloalkyl $C_{1-4}$ alkyl or phenyl $C_{1-4}$ alkyl halide, such as a chloride, bromide or iodide.

The compounds of the present invention and their pharmaceutically acceptable salts form solvates with pharmaceutically acceptable solvents, for example hydrates.

Of the compounds exemplified hereinafter those that are preferred are the compounds of Examples 2, 18, 20, 21c, 21f, 21h, 21i, 25 and 37c.

The present invention also provides a process for preparing a compound of formula (I), or a pharmaceutically acceptable salt, quaternised derivative, N-oxide or solvate thereof, which comprises reacting a compound of formula (II), or an acid addition salt thereof:

$$R_1-Ar-(CH_2)_a-X-(CH_2)_b-NH_2 \qquad (II)$$

wherein $R_1$, Ar, a, X and b are as defined hereinbefore, with a compound of formula (III):

$$Het\text{-}L_1 \qquad (III)$$

wherein Het is as defined hereinbefore and $L_1$ is a leaving group; in the case where n in the resulting compound of formula (I) is 0, optionally oxidising the compound to obtain another compound of formula (I) wherein n is an integer 1 or 2; optionally converting $R_1$, $R_4$, $R_5$, $R_6$ or $R_8$ in the resulting compound of formula (I) into another $R_1$, $R_4$, $R_5$, $R_6$ or $R_8$; and optionally forming a pharmaceutically acceptable salt, quaternised derivative, N-oxide or solvate thereof.

The leaving group ($L_1$) is such as to be displaceable by the compound of formula (II). Examples of the leaving group ($L_1$) include halogen, such as chloro and bromo, $C_{1-4}$ alkoxy, such as methoxy and ethoxy, $C_{1-4}$ alkylthio, such as methylthio and ethylthio, $C_{1-4}$ alkylsulphinyl, such as methylsulphinyl, and thiol. Preferred examples of the leaving group ($L_1$) include chloro, methoxy, ethoxy, methylthio and thiol. Particularly preferred examples are chloro and ethoxy.

The reaction may be carried out in a solvent, such as dichloromethane, toluene, 2-methoxy-ethanol, methanol, isopropanol, dimethylformamide or dimethoxyethane/ methanol, at room temperature or above, for example, at reflux temperature, optionally in the presence of an acid acceptor, such as triethylamine.

In the case where an acid addition salt of a compound of formula (II) is used in the reaction, it is preferred that the leaving group ($L_1$) is halogen and that an acid acceptor is present during the course of the reaction. It is even more preferred, however, that the compound of formula (II) is used not in the form of an acid addition salt but in the form of its free base.

In the case where n in the resulting compound of formula (I) is 0, the optional oxidation of the compound to obtain another compound of formula (I), wherein n is an integer 1 or 2, may be carried out with a metal, such as sodium, periodate or with one equivalent of m-chloroperbenzoic acid, when n is 1, or with an excess of m-chloroperbenzoic acid, when n is 2. It is preferred, however, that such oxidation is not carried out on a compound of formula (I), wherein X is -S-. It is even more preferred that any oxidation of this kind is carried out at an earlier stage in the process of the invention rather than on a compound of formula (I).

Examples of an optional conversion of $R_1$ in the resulting compound of formula (I) into another $R_1$ include the optional conversion of an aminomethylene group of formula (a), wherein $R_2$ and $R_3$ are hydrogen, into another aminomethylene group of formula (a), wherein $R_2$ or $R_3$ are $C_{1-4}$ alkyl. Such a conversion may be carried out by direct alkylation or by reductive alkylation.

Examples of an optional conversion of $R_4$ and/or $R_5$ in the resulting compound of formula (I) into another $R_4$ and/or $R_5$ include the optional conversion of

hydrogen into nitro by nitration, nitro into amino by reduction, halogen into amino by amination, and amino, aminocarbonyl, aminocarbonylamino, amino-thiocarbonylamino and aminosulphonyl into amino, aminocarbonyl, aminocarbonylamino, amino-thiocarbonylamino and aminosulphonyl, the amino group or the amino moiety of the amino-containing groups being substituted by one or two $C_{1-6}$ alkyl, by alkylation or by reductive alkylation. A particularly useful example of an optional conversion of $R_4$ or $R_5$ in the resulting compound of formula (I) into another $R_4$ or $R_5$ is the optional conversion of nitro into amino by reduction, for example, with iron powder and glacial acetic acid.

Examples of an optional conversion of $R_6$ or $R_8$ in the resulting compound of formula (I) into another $R_6$ or $R_8$ include the optional conversion of hydrogen into $C_{1-6}$ alkyl by alkylation.

The optional formation of a pharmaceutically acceptable salt, quaternised derivative, N-oxide or solvate may be carried out conventionally.

The compounds of formula (II) are known, or can be prepared analogously to the preparation of known compounds, for example, from U.S. Patents 3 905 984, 4 022 797, 4 053 473, 4 128 658, 4 165 378 and 4 220 654.

The compounds of formula (III), wherein Het is a bicyclic heteroaryl group of formula (b), may be prepared from a compound of formula (IV):

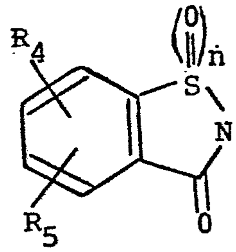

(IV)

wherein $R_4$, $R_5$ and n are as defined hereinbefore, by reaction with a halogenating agent and optionally converting the halogen leaving group ($L_1$) in the resulting compound of formula (III) into another leaving group ($L_1$), or, in the case where $L_1$ is $C_{1-4}$ alkoxy, by reaction with tri $C_{1-4}$ alkoxonium tetrafluoroborate.

Examples of a halogenating agent include thionyl halide, phosphorus oxyhalide and phosphorus pentahalide. A preferred example is phosphorus pentachloride.

The reaction of a compound of formula (IV) and a halogenating agent may be carried out either by heating the reactants in neat form in a high boiling inert solvent, such as decalin, or in an excess of the halogenating agent as solvent optionally in the presence of a tertiary base, such as 2,6-lutidine, to a temperature of 100-180°C as appropriate.

The optional conversion of the halogen leaving group ($L_1$) in the resulting compound of formula (III) into another leaving group ($L_1$) may be carried out in accordance with conventional methods. For example, the resulting compound of formula (III) may be reacted with an appropriate $C_{1-4}$ alcohol or $C_{1-4}$ thioalcohol

optionally in the presence of an acid halide acceptor, such as pyridine, or in an inert solvent, such as dichloromethane. It is particularly preferred that the compound of formula (IV) is reacted with phosphorus pentachloride and that the resulting compound of formula (III), wherein $L_1$ is chloro, is optionally reacted with a $C_{1-4}$ alcohol, in particular methanol or ethanol.

The compounds of formula (IV) are either known, for example, from U.S. Patent 4 104 387 or Japanese Patents 590 963 and 656 470, or may be prepared in an analogous manner to the preparation of known compounds.

The compounds of formula (III), wherein Het is a bicyclic heteroaryl group of formula (c), may be prepared by reacting a compound of formula (V):

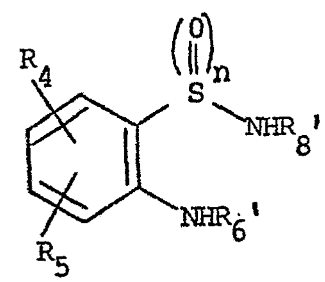

(V)

wherein one of $R_6$' and $R_8$' is hydrogen or $C_{1-6}$ alkyl and the other is hydrogen, and $R_4$, $R_5$ and n are as defined hereinbefore, with urea or thiourea, and, in the case of urea reacting the resulting compound of formula (VI):

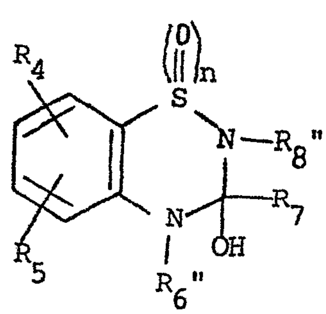

(VI)

wherein one of $R_6''$ and $R_8''$ is hydrogen or $C_{1-6}$ alkyl and the other together with $R_7$ is a bond, and $R_4$, $R_5$ and n are as defined hereinbefore, with a halogenating agent; optionally converting the halogen or thiol leaving group ($L_1$) in the resulting compound of formula (III) into another leaving group ($L_1$); and optionally converting one of $R_6$ and $R_8$, when hydrogen, in the resulting compound of formula (III) into one of the other substituents defined hereinbefore.

The reaction of a compound of formula (V) with either urea or thiourea may be carried out following the procedure of Parke and Williams (J. Chem. Soc., 1950, 1760).

Examples of a halogenating agent include those mentioned hereinbefore in relation to their reaction with a compound of formula (IV).

The reaction of a compound of formula (VI) with a halogenating agent may be carried in the same manner as the reaction between a compound of formula (IV) and a halogenating agent, as described hereinbefore.

The optional conversion of the halogen leaving group ($L_1$) in the resulting compound of formula (III) into another leaving group ($L_1$) may be carried out as described hereinbefore.

The optional conversion of the thiol leaving group ($L_1$) in the resulting compound of formula (III) into another leaving group ($L_1$) may be carried out in accordance with conventional methods. For example, the resulting compound of formula (III) may be reacted with an alkylating agent so as to obtain the corresponding compound of formula (III), wherein $L_1$ is $C_{1-4}$ alkylthio.

Examples of an optional conversion of one of $R_6$ and $R_8$, when hydrogen, in the resulting compound of formula (III) into one of the other substituents defined hereinbefore include those examples described hereinbefore in relation to the optional conversion of $R_6$ and $R_8$ in a resulting compound of formula (I).

The compounds of formula (V) are known or may be prepared in an analogous manner to the preparation of known compounds.

The present invention provides a second process for preparing a compound of formula (I), wherein X is $-S-$, $-O-$, $-\overset{O}{\overset{\|}{S}}-$ or $-NH$, or a pharmaceutically acceptable salt, quaternised derivative, N-oxide or solvate thereof, which comprises reacting a compound of formula (VII):

$$R_1-Ar-(CH_2)_a-L_2 \qquad (VII)$$

wherein $R_1$, Ar and a are as defined hereinbefore and $L_2$ is a leaving group, with a compound of formula (VIII):

$$H-X'-(CH_2)_b-NH-Het \qquad (VIII)$$

wherein b and Het are as defined hereinbefore and X' is $-S-$, $-O-$, $-\overset{O}{\overset{\|}{S}}-$ or $-NH-$; in the case where n in the resulting compound of formula (I) is O, optionally

oxidising the compound to obtain another compound of formula (I) wherein n is an integer 1 or 2; optionally converting $R_1$, $R_4$, $R_5$, $R_6$ or $R_8$ in the resulting compound of formula (I) into another $R_1$, $R_4$, $R_5$, $R_6$ or $R_8$; and optionally forming a pharmaceutically acceptable salt, quaternised derivative, N-oxide or solvate thereof.

The leaving group ($L_2$) is such as to be displaceable by the compound of formula (VIII). Examples of the leaving group ($L_2$) include halogen, such as chloro and bromo, hydroxy, and mesyloxy and tosyloxy.

The reaction between the compounds of formulae (VII) and (VIII) may be carried out conventionally using either a polar protic or aprotic solvent, in the presence of an acid acceptor, such as triethylamine or a metal carbonate, or in the presence of a strong acid, such as hydrochloric acid.

The optional oxidation may be carried out as described hereinbefore.

Examples of an optional conversion of $R_1$, $R_4$, $R_5$, $R_6$ or $R_8$ include those described hereinbefore.

The optional formation of a pharmaceutically acceptable salt, quaternised derivative, N-oxide or solvate may be carried out as described hereinbefore.

The compounds of formula (VII) are either known, for example, from U.K. Patent Application 81 14 575, Spanish Patent 506 422 and J. Chem. Soc., 1958, 4728 to 4731, or may be prepared in an analogous manner to the preparation of known compounds.

The compounds of formula (VIII) may be prepared by reacting a compound of formula (III), as defined hereinbefore, with a compound of formula (IX):

$$H{-}X'{-}(CH_2)_b{-}NH_2 \qquad (IX)$$

wherein b and X' are as defined hereinbefore.

The reaction between the compounds of formulae (III) and (IX) may be carried out under the conditions described hereinbefore in relation to the reaction between the compounds of formulae (II) and (III).

The compounds of formula (IX) are known compounds.

The present invention provides a third process for preparing a compound of formula (I), wherein $R_1$ is an aminomethylene group of formula (a), as defined hereinbefore, or a pharmaceutically acceptable salt, quaternised derivative, N-oxide or solvate thereof, which comprises reacting a compound of formula (X):

$$L_3CH_2{-}Ar{-}(CH_2)_a{-}X{-}(CH_2)_b{-}NH{-}Het \qquad (X)$$

wherein Ar, a, X, b and Het are as defined hereinbefore and $L_3$ is a leaving group, with an amine of formula (XI):

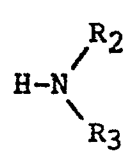

(XI)

wherein $R_2$ and $R_3$ are as defined hereinbefore; in the case where n in the resulting compound of formula (I) is 0, optionally oxidising the compound to obtain another compound of formula (I) wherein n is an integer 1 or 2; optionally converting $R_1$, $R_4$, $R_5$, $R_6$ or $R_8$ in the resulting compound of formula (I) into another $R_1$, $R_4$, $R_5$, $R_6$ or $R_8$; and optionally forming a pharmaceutically acceptable salt, quaternised derivative, N-oxide or solvate thereof.

The leaving group ($L_3$) is such as to be displaceable by the amine of formula (XI). Examples of the leaving group ($L_3$) include hydroxy, chloro, bromo, mesyloxy and tosyloxy.

The reaction between the compound of formula (X) and the amine of formula (XI) may be carried out under the conditions described hereinbefore in relation to the reaction between the compounds of formulae (VII) and (VIII).

The optional oxidation may be carried out as described hereinbefore.

Examples of an optional conversion of $R_1$, $R_4$, $R_5$, $R_6$ and $R_8$ include those described hereinbefore.

The optional formation of a pharmaceutically acceptable salt, quaternised derivative, N-oxide, or solvate may be carried out as described hereinbefore.

The compounds of formula (X) may be prepared by reacting a compound of formula (III), as defined hereinbefore, with a compound of formula (XII):

$$L_3CH_2\text{-}Ar\text{-}(CH_2)_a\text{-}X\text{-}(CH_2)_b\text{-}NH_2 \qquad (XII)$$

wherein $L_3$, Ar, a, X and b are as defined hereinbefore.

The reaction between the compounds of formulae (III) and (XII) may be carried out under the conditions described hereinbefore in relation to the reaction between the compounds of formulae (II) and (III).

The compounds of formula (XII) may be prepared by reacting a compound of formula (XIII):

$$L_3'CH_2-Ar-(CH_2)_aL_4 \qquad (XIII)$$

wherein Ar and a are as defined hereinbefore, $L_3'$ is hydroxy or protected hydroxy and $L_4$ is a preferential leaving group, with a compound of formula (XIV):

$$HX-(CH_2)_b-NR_{11}R_{12} \qquad (XIV)$$

wherein X and b are as defined hereinbefore and $R_{11}$ and $R_{12}$ are both hydrogen, or one of $R_{11}$ and $R_{12}$ is hydrogen and the other is a protecting group, or both

of $R_{11}$ and $R_{12}$ together are a protecting group; in the case where in the resulting compound $L_3$ is protected hydroxy, removing the protecting group; optionally converting the hydroxy leaving group ($L_3$) in the resulting compound into another leaving group ($L_3$); and in the case where in the resulting compound one of $R_{11}$ and $R_{12}$ or both of $R_{11}$ and $R_{12}$ together are a protecting group, removing the protecting group.

Examples of the group ($L_3'$), when protected hydroxy, include tetrahydropyranyl.

The preferential leaving group ($L_4$) is such as to be displaceable (in preference to the group ($L_3'$)) by the compound of formula (XIV). Examples of the preferential leaving group ($L_4$) include chloro, bromo, hydroxy, mesyloxy and tosyloxy. When $L_4$ is hydroxy and a is other than 1, it is preferred that $L_3'$ is protected hydroxy.

In the case where one of $R_{11}$ and $R_{12}$ is hydrogen and the other is a protecting group, examples of a protecting group include $C_{1-4}$ alkanoyl.

In the case where both of $R_{11}$ and $R_{12}$ together are a protecting group, examples of a protecting group include phthalimidyl.

The reaction between the compounds of formulae (XIII) and (XIV) may be carried out under the conditions described hereinbefore in relation to the reaction between the compounds of formulae (VII) and (VIII).

The removal of a protecting group from the resulting comound, wherein the group ($L_3'$) is protected hydroxy, may be carried out conventionally.

0105732

The optional conversion of the hydroxy leaving group ($L_3$) into another leaving group ($L_3$) may be carried out as described hereinbefore in relation to the leaving group ($L_1$).

The removal of a protecting group from the resulting compound, wherein one of or both together of $R_{11}$ and $R_{12}$ are a protecting group, may be carried out conventionally.

The present invention provides a fourth process for preparing a compound of formula (I), wherein $R_1$ is an aminomethylene group of formula (a), as defined hereinbefore, or a pharmaceutically acceptable salt, quaternised derivative, N-oxide or solvate thereof, which comprises reacting a compound of formula (XV):

$$H\text{-}Ar\text{-}(CH_2)_a\text{-}X\text{-}(CH_2)_b\text{-}NH\text{-}Het \qquad (XV)$$

wherein Ar, a, X, b and Het are as defined hereinbefore, with formaldehyde and a compound of formula (XI), as defined hereinbefore; in the case where n in the resulting compound of formula (I) is 0, optionally oxidising the compound to obtain another compound of formula (I) wherein n is an integer 1 or 2; optionally converting $R_1$, $R_4$, $R_5$, $R_6$ or $R_8$ in the resulting compound of formula (I) into another $R_1$, $R_4$, $R_5$, $R_6$ or $R_8$; and optionally forming a pharmaceutically acceptable salt, quaternised derivative, N-oxide or solvate thereof.

The reaction between the compounds of formulae (XV) and (XI) and formaldehyde is a Mannich reaction and may be carried out under conventional Mannich rection conditions, for example, in aqueous media in the presence of a strong acid, such as hydrochloric acid.

The optional oxidation may be carried out as described hereinbefore.

Examples of an optional conversion of $R_1$, $R_4$, $R_5$, $R_6$ or $R_8$ include those described hereinbefore.

The optional formation of a pharmceutically acceptable salt, quaternised derivative, N-oxide or solvate may be carried out as described hereinbefore

The compound of formula (XV) may be prepared by reacting a compound of formula (III), as defined hereinbefore, and a compound of formula (XVI):

$$H\text{-}Ar\text{-}(CH_2)_a\text{-}X\text{-}(CH_2)_b\text{-}NH_2 \qquad (XVI)$$

wherein Ar, a, X and b are as defined hereinbefore.

The reaction between the compounds of formulae (III) and (XVI) may be carried out under conditions described hereinbefore in relation to the reaction between the compounds of formulae (II) and (III).

The compounds of formula (XVI) may be prepared by reacting a compound of formula (XVII):

$$H\text{-}Ar\text{-}(CH_2)_a\text{-}L_5 \qquad (XVII)$$

wherein Ar and a are as defined hereinbefore and $L_5$ is a leaving group, with a compound of formula (XIV), as defined hereinbefore; in the case where in the resulting compound one of $R_{11}$ and $R_{12}$ or both of $R_{11}$ and $R_{12}$ together are a protecting group, removing the protecting group.

The leaving group ($L_5$) is such as to be displaceable by the compound of formula (XIV). Examples of the leaving group ($L_5$) include chloro, bromo, hydroxy, mesyloxy and tosyloxy.

The reaction between the compounds of formulae (XVII) and (XIV) may be carried out under the conditions as described hereinbefore in relation to the reaction between the compounds of formulae (VII) and (VIII).

The compounds of formulae (III), (VIII), (X) and (XV) are novel intermediates and represent part of the present invention. A sub-class of such novel intermediates is of formulae (III), (VIII), (X) and (XV), wherein Het is of formula (b), $R_4$ being hydrogen and $R_5$ being as defined hereinbefore in the 4-position, or is of formula (c). A preferred sub-class is of formulae (III), (VIII), (X) and (XV), wherein Het is of formula (b), $R_4$ being hydrogen and $R_5$ being nitro or amino in the 4-position.

As mentioned hereinbefore, the compounds of formula (I), and their pharmaceutically acceptable salts, quaternised derivatives, N-oxides and solvates thereof, are histamine $H_2$-receptor antagonists and, therefore, are able to inhibit gastric acid secretion. In this way, the compounds may be used in the treatment or prophylaxis of any disorder caused or exacerbated by excess gastric acid secretion, such as peptic ulcers.

Accordingly, the present invention also provides a pharmaceutical composition, which comprises a compound of formula (I), as defined hereinbefore, or a pharmaceutically acceptable salt, quaternised derivative, N-oxide or solvate thereof, and a pharmaceutically acceptable carrier.

The composition of the present invention may be formulated for administration by any route, although in general oral administration is preferred. The composition may be in the form of a tablet, capsule, powder, granule, lozenge, suppository, reconstitutable powder, or a liquid preparation, such as an oral or sterile parenteral solution or suspension.

In order to obtain consistency of administration it is preferred that a composition of the invention is in the form of a unit dose.

Unit dose presentation forms for oral adminstration may take the form of a tablet or a capsule and may contain conventional excipients such as a binding agent, for example syrup, acacia, gelatin, sorbitol, tragacanth, or polyvinyl-pyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions of the present invention may be prepared by conventional methods of blending, filling, tabletting or the like. Repeated blending operations may be used to distribute the active agent throughout those compositions employing large quantities of fillers.

Such operations are of course conventional in the art. The tablets may be coated according to methods well known in normal pharmaceutical practice.

A particular composition of the invention is a tablet containing the required amount of a compound of the invention in the form of a powder or granulate compressed in intimate mixture with a lubricant, such as magnesium stearate, a filler, such as microcrystalline cellulose, and a disintegrant, such as sodium starch glycollate.

Oral liquid preparations may be in the form of, for example, an emulsion, syrup, or elixir, or may be presented as a dry product for reconstitution with water or other suitable vehicle before use. Such liquid preparations may contain conventional additives such as a suspending agent, for example sorbitol, syrup, methyl cellulose, gelatin, hydroxyethylcellulose, carboxymethylcellulose, aluminium stearate gel, hydrogenated edible fats; an emulsifying agent, for example lecithin, sorbitol monooleate, or acacia; a non-aqueous vehicle (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and a buffering agent can be dissolved in the vehicle. To enhance the stability, the

composition can be frozen after filling into the vial and the water removed under vacuum. Parenteral suspensions are prepared in substantially the same manner, except of course that the compound is suspended in the vehicle instead of being dissolved, and sterilization cannot be accomplished by filtration. However, the compound can be sterilized by exposure to ethylene oxide before suspending in the sterile vehicle. Advantageously, a surfactant or wetting agent is included in the composition to facilitate uniform distribution of the compound.

When appropriate the composition of the invention may be presented as a suppository for rectal or vaginal administration. Suitable unit dose forms include tablets, capsules and powders in sachets or vials, and preferred forms include shaped oral unit doses, such as tablets and capsules.

The compositions may contain from 0.1% to 99% by weight, preferably from 10-60% by weight, of the active material, depending on the method of administration.

It will be appreciated of course that the quantity of the compound administered to the sufferer each day will depend on the usual factors such as the severity of the disease, and the weight of the sufferer and on the dose-response characteristics of the particular active ingredient. Suitably however it is believed that between 0.1 to 25 mg/kg/day of the compound will be administered to achieve satisfactory therapy. Such administration is conveniently effected with repeated dosing throughout the day of the composition in unit dose form.

By way of example, unit dose compositions will suitably contain from 5 to 2000 mg, more preferably from 10 to 1000 mg of the active ingredient.

The invention also provides a method of treatment or prophylaxis of disorders, such as peptic ulcers, in mammals including humans, caused or exacerbated by excess gastric acid secretion, which method comprises the administration to the mammal of an effective amount of the compound of formula I, as hereinbefore defined, or a pharmaceutically acceptable salt, quaternised derivative, N-oxide or solvate thereof.

The invention further provides a compound of formula (I), as hereibefore defined, or a pharmaceutically acceptable salt, quaternised derivate, N-oxide or solvate thereof for use in the treatment or prophylaxis of mammals, such as humans, in particular in the treatment or prophylaxis of disorders caused or exacerbated by excess gastric acid secretion, such as peptic ulcers.

The following Examples illustrate the compounds of the present invention.

Description 1

Preparation of 3-chloro-4-nitro-1,2-benzoisothiazole-1,1-dioxide

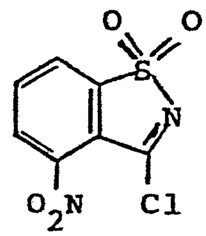

(D1a)

An intimate mixture of 4-nitrosaccharin [0.50g, prepared as described by G.H. Hamor, J. Amer. Pharm. Soc., 49, 280 (1960)] and phosphorus pentachloride was heated at 180°C for 1.5 hours. The phosphorus oxychloride formed was removed by distillation and the dark residue was partitioned between water (50ml) and dichloromethane (2 x 50ml). The combined organic phase was washed with brine (2 x 70ml) and dried over magnesium sulphate. Removal of solvent in vacuo gave an amorphous orange residue (0.43g) which was used without further purification. A sample was triturated under ether to yield a white solid mpt. 203-5°C

Prepared analogously were:-

3-chlorobenzoisothiazole-1,1-dioxide (D1b)
3,4-dichlorobenzoisothiazole-1,1-dioxide (D1c)
3-chloro-5-nitrobenzoisothiazole-1,1-dioxide (D1d)
3-chloro-6-nitrobenzoisothiazole-1,1-dioxide (D1e)
3-chloro-7-nitrobenzoisothiazole-1,1-dioxide (D1f)
3,6-dichlorobenzoisothiazole-1,1-dioxide (D1g)
3-chloro-6-dimethylsulphamoyl-benzoisothiazole-1,1-dioxide (D1h)
3-chloro-4-methylbenzoisothiazole-1,1-dioxide (D1i)
3-chloro-4-iodobenzoisothiazole-1,1-dioxide (D1j)
3-chloro-4-cyanobenzoisothiazole-1,1-dioxide (D1k)

Description 2a

3-Ethoxy-4-nitro-benzoisothiazole-1,1-dioxide

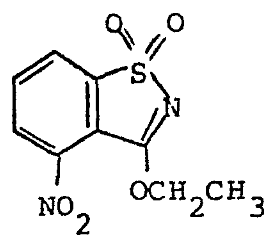

(D2a)

Ethanol (100ml) was added to a solution of 3-chloro-4-nitro-benzoisothiazole-1,1-dioxide (0.86g) in dichloromethane (110ml). The resulting solution was allowed to stand overnight at room temperature. Removal of the solvent *in vacuo* gave an amorphous brown solid, which was triturated under ether (100ml) to give the title compound as a white solid (0.82g), mpt 190-191°C.

'H-NMR $CDCl_3$-$d^6$-DMSO
$\delta$ = 1.33 (t, 3H)
$\delta$ = 4.47 (q, 2H)
$\delta$ = 7.95-8.34 (m, 3H)

Description 2b

3-Ethoxy-6-nitro-benzoisothiazole-1,1-dioxide

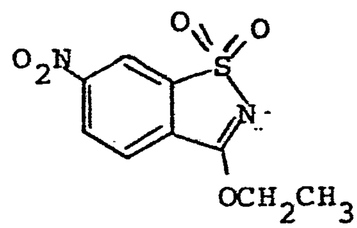

(D2b)

Following the procedure outlined in Description 2a, 3-chloro-6-nitro-benzoisothiazole-1,1-dioxide (4.2g) was converted to the title compound (4.3g).

'H-NMR $CDCl_3$

$\delta$ = 1.56 (t, 3H)

$\delta$ = 4.66 (q, 2H)

$\delta$ = 7.96 (d, 1H)

$\delta$ = 8.4-8.75 (m, 2H)

Description 2c

3-Ethoxy-5-nitro-benzoisothiazole-1,1-dioxide

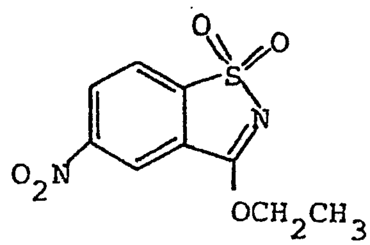

(D2c)

Following the procedure outlined in Description 2a, 3-chloro-5-nitro-benzoisothiazole-1,1-dioxide (2.9g) was converted to the title compound (2.4g)

mp 151-4°

$^1H$-NMR $CDCl_3$-$d^6$ DMSO

$\delta$ = 1.55 (t, 3H)
$\delta$ = 4.62 (q, 2H)
$\delta$ = 8.08 (d, 1H, J = 8.5Hz)
$\delta$ = 8.35-8.7 (m, 2H)

Description 2d

3-Ethoxy-4-methyl-benzoisothiazole-1,1-dioxide

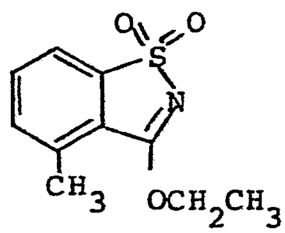

(D2d)

Following the procedure outlined in Description 2a, 3-chloro-4-methyl-benzoisothiazole-1,1-dioxide was converted to the title compound.

'H-NMR $d_6$-DMSO

$\delta$ = 1.52 (t, 3H)
$\delta$ = 2.65 (s, 3H)
$\delta$ = 4.60 (q, 2H)
$\delta$ = 7.5-8.15 (m, 3H)

Description 2e

3-Ethoxy-6-dimethylsulphamoylbenzoisothiazole-1,1-dioxide

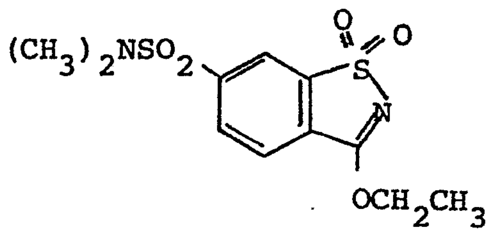

(D.2e)

Following the procedure outlined in Description 2a, 3-chloro-6-dimethylsulphamoylbenzoisothiazole-1,1-dioxide (D1h) was converted to the title compound.

$^1$H-NMR $CDCl_3 + CD_3OD$

δ = 1.55 (t, 3H)
δ = 2.76 (s, 6H)
δ = 4.66 (q, 2H)
δ = 7.7-8.3 (m, 3H)

The following 3-ethoxy-6-sulphamoylbenzoisothiazole-1,1-dioxides were prepared similarly.

3-Ethoxy-6-methylsulphamoylbenzoisothiazole-1,1-dioxide (D.2f)

Description 3a

4-Amino-3-ethoxy-benzoisothiazole-1,1-dioxide

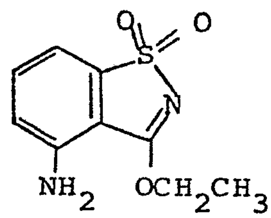

(D3a)

A solution of 3-ethoxy-4-nitro-benzoisothiazole-1,1-dioxide (0.81g) in ethanol (250ml) in the presence of 5% Pd-carbon (0.2g) was hydrogenated at atmospheric pressure and ambient temperature until the theoretical volume of hydrogen had been taken up.

The mixture was filtered through Kieselguhr and the residue washed thoroughly with methanol. Removal of solvent *in vacuo* gave the title compound as a pale yellow solid (0.63g) mpt 213-216°C (dec).

'H-NMR $d^6$-DMSO, $CDCl_3$

$\delta$ = 1.43 (t, 3H)

$\delta$ = 4.48 (q, 2H)

$\delta$ = 6.26 (br.s. 2H)

$\delta$ = 6.7-6.96 (m, 2H)

$\delta$ = 7.13-7.5 (m, 1H)

Description 3b

6-Amino-3-ethoxy-benzoisothiazole-1,1-dioxide

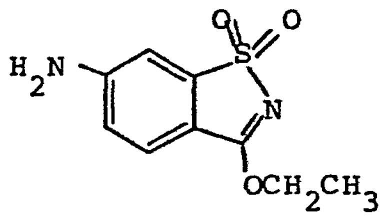

(D3b)

Following the procedures outlined in Description 3a, 3-ethoxy-6-nitro-benzoisothiazole-1,1-dioxide (4.3g) was converted to the title compound (2,4g).

'H-NMR $d^6$-DMSO-$CDCl_3$

$\delta$ = 1.42 (t, 3H)
$\delta$ = 4.45 (d, 2H)
$\delta$ = 5.4-6.9 (br.s. 2H)
$\delta$ = 6.5-7.0 (m, 2H)
$\delta$ = 7.3 (d, 1H)

Mass spectrum $C_9H_{10}N_2O_3S$ Required 226.0412
Observed 226.0411

Description 3c

5-Amino-3-ethoxy-benzoisothiazole-1,1-dioxide

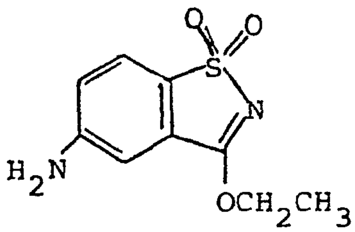

(D3c)

Following the procedures outlined in Description 3a 3-ethoxy-5-nitro-benzoisothiazole-1,1-dioxide (2.3g) was converted to the title compound (0.95g).

mp 154-6°

$^{1}$H-NMR CDCl$_3$ - d$^6$ DMSO

$\delta$ = 1.43 (t, 3H)
$\delta$ = 4.45 (q, 2H)
$\delta$ = 6.8-7.1 (m, 2H)
$\delta$ = 7.52 (d, 1H, J = 9Hz)
$\delta$ = 8-8.4 (brs, 2H exchangeable with $D_2O$)

Description 4a

6-Acetamido-3-ethoxy-benzoisothiazole-1,1-dioxide

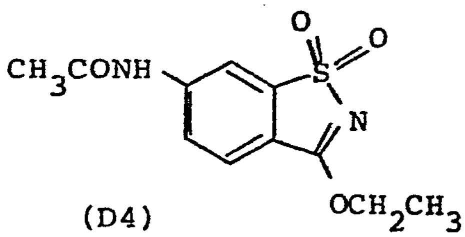

(D4)

A solution of 6-amino-3-ethoxy-benzoisothiazole-1,1-dioxide (0.23g) and acetic anhydride (0.1ml) in pyridine (1ml) was stirred at room temperature for 15 hrs. Concentration and trituration under ether/petrol (1:1) gave the title compound (0.24g).

'H-NMR $d^6$DMSO - $CDCl_3$

δ = 1.50 (t, 3H)

δ = 2.17 (s, 3H)

δ = 4.62 (q, 2H)

δ = 7.5-8.0 (m, 2H)

δ = 8.37 (d, 1H)

δ = 10.5 (br.s. 1H)

Mass spectrum $C_{11}H_{12}N_2O_4S$ Required 268.0518

Observed 268.0511

Description 4b

6-Methylcarbamoylamino-3-ethoxy-benzoisothiazole-1,1-dioxide

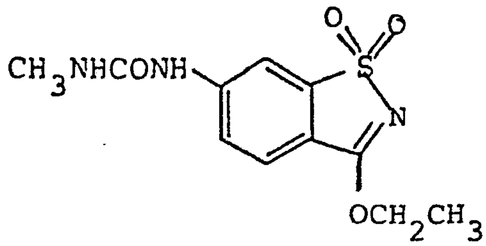

(D4b)

Following the procedures outlined in Description 4a, 6-amino-3-ethoxy-benzoisothiazole-1,1-dioxide (0.23g) was reacted with methyl isocyanate for 3 days to give the title compound.

$^1$H-NMR  $CDCl_3$ + $d_6$ DMSO

$\delta$ = 1.5 (t, 3H)
$\delta$ = 2.7, 2.76 (2S, 3H)
$\delta$ = 4.53 (q, 2H)
$\delta$ = 7.3-8.1 (m, 2H)
$\delta$ = 8.16 (brs, 1H)

Mass spectrum  $C_{11}H_{13}N_3O_3S$  Required 283.0627
Observed 283.0626

Description 4c

6-Methanesulphamoylamino-3-ethoxy-benzoisothiazole-1,1-dioxide

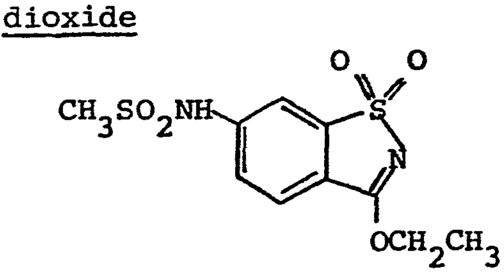

(D.4c)

Following the procedures outlined in Description 4a, 6-amino-3-ethoxy-benzoisothiazole-1,1-dioxide was reacted with methane sulphonyl chloride to give the title compound.

Description 4d

6-Propionamido-3-ethoxy-benzoisothiazole-1,1-dioxide

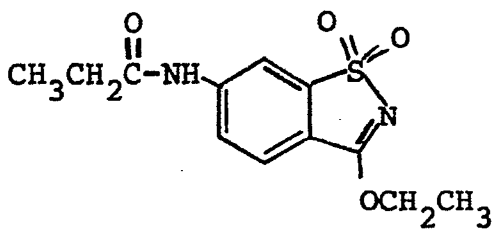

(D.4d)

Following the procedures outlined in Description 4a, 6-amino-3-ethoxy-benzoisothiazole-1,1-dioxide was reacted with propionic anhydride to give the title compound.

mp 188-92°

$^1$H-NMR $CDCl_3 + CD_3OD$

δ = 1.25 (t, 3H)
δ = 1.55 (t, 3H)
δ = 2.47 (q, 2H)
δ = 4.65 (q, 2H)
δ = 7.35-8.25 (m, 3H)

Mass spectrum $C_{12}H_{14}N_2O_4S$ Required 282.0674
Observed 282.0682

Description 4e

6-Formamido-3-ethoxy-benzoisothiazole-1,1-dioxide

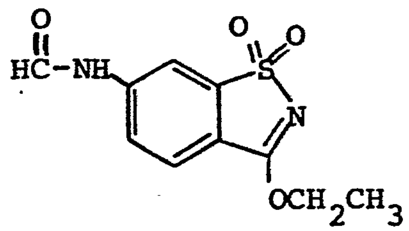

(D.4e)

Following the procedures outlined in Description 4a, 6-amino-3-ethoxy-benzoisothiazole-1,1-dioxide was reacted with formic-acetic anhydride to give the title compound.

mp 188-94°

$^1$H-NMR $CDCl_3 + CD_3OD$

$\delta$ = 1.52 (t, 3H)
$\delta$ = 4.65 (q, 2H)
$\delta$ = 7.6-8.1 (m, 2H)
$\delta$ = 8.25 (brs, 1H)
$\delta$ = 8.42 (s, 1H)

Mass spectrum $C_{10}H_{10}N_2O_4S$ Required 254.0362
Observed 254.0366

Description 5

5,6-Dimethoxy-3-ethoxy-benzoisothiazole-1,1-dioxide

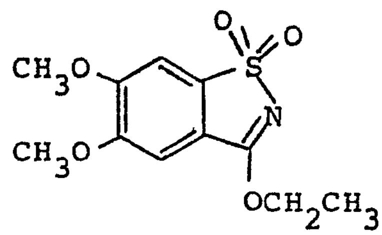

(D5)

To a solution of 5,6-dimethoxysaccharin (0.8g) in dichloromethane (100ml) was added triethyloxonium tetrafluoroborate (3.5ml, 1M soln in $CH_2Cl_2$) and the solution stirred at room temperature for 3hrs. After extraction with aqueous sodium bicarbonate, the organic phase was dried and concentrated. Purification by column chromatography (silica, chloroform) gave the title compound (0.2g).

'H-NMR $CDCl_3$

$\delta$ = 1.5 (t, 3H)

$\delta$ = 3.92 (s, 6H)

$\delta$ = 4.53 (q, 2H)

$\delta$ = 6.97 (s, 1H)

$\delta$ = 7.23 (s, 1H)

Description 6

Preparation of 6-dimethylsulphamoyl saccharin

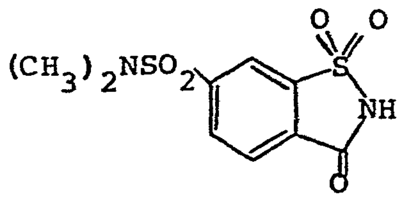

(D6)

A suspension of 6-aminosaccharin (1g) in glacial acetic acid (8ml), conc. hydrochloric acid (4 ml) and water (3ml) was diazotised with a solution of sodium nitrite (0.37g) in water (1ml) at 0-10° for 1h. The diazonium salt solution was poured into a solution of cupric chloride (1g) in water (2.5ml), glacial acetic acid (20ml) and sulphur dioxide (12g) and the mixture was stirred at room temperature for 1h. On pouring into ice, the crude sulphonyl chloride which precipitated was collected and dried (0.5g, mp 191-3°).

The sulphonyl chloride was dissolved in alcoholic dimethylamine (33% v/v, 15ml) and stirred overnight at room temperature. On evaporation of the solvent, water trituration yielded the title compound (0.4g) mp 198-200°.

'H - NMR $d^6$ DMSO

$\delta$ = 2.70 (s,6H)

$\delta$ = 8.25 (br s, 2H)

$\delta$ = 8.40 (br s, 1H)

mass spectrum $C_9H_{10}N_2O_5S_2$ Required 290.0032

Observed 290.0040

Description 7

N-[3-(3-Piperid-1-ylmethylphenoxy)propyl]-1,2-benzoisothiazole-3-amine

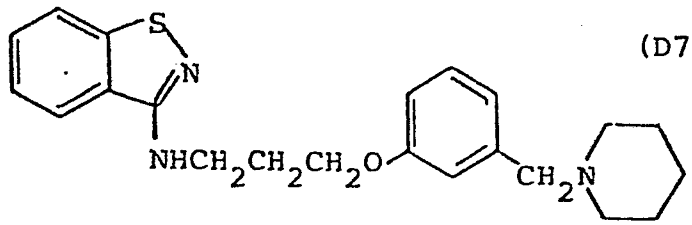

(D7)

was prepared by the method of Example 13 using DMF as the solvent and potassium carbonate as the base with heating under reflux for 1 hour.

$^1$H-NMR CDCl$_3$ + D$_2$O

$\delta$ = 1.2-1.8 (m, 6H)
$\delta$ = 2.0-2.6 (m, 6H)
$\delta$ = 3.43 (s, 2H)
$\delta$ = 3.80 (t, 2H)
$\delta$ = 4.15 (t, 2H)
$\delta$ = 6.7-7.9 (m, 8H)

| | | |
|---|---|---|
| Mass spectrum $C_{22}H_{27}N_3OS$ | Required | 381.1874 |
| | Observed | 381.1875 |

Description 8

Preparation of 6-iodo-3-ethoxy-benzoisothiazole-1,1-dioxide

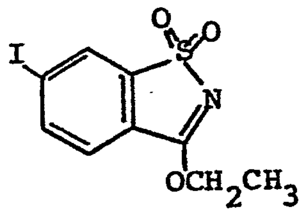

(D.8)

6-Amino-3-ethoxybenzoisothiazole-1,1-dioxide (D.3b) (0.25g) was diazotised with sodium nitrite (0.21g) in conc. sulphuric acid (5ml) at 0-10°C for 2h. After pouring into an aqueous (50ml) solution of potassium iodide (1g), the black suspension was treated with sodium metabisulphite until an orange precipitate remained. The solid was collected, washed with water and dried (0.26g, mp 185-90°)

| | | |
|---|---|---|
| $^1$H NMR | δ = 1.28 | (t, 3H) |
| | δ = 4.34 | (q, 2H) |
| | δ = 7.12 | (d, 1H) |
| | δ = 7.75 | (dd, 1H) |
| | δ = 7.90 | (brs, 1H) |

DESCRIPTION 9

$N^3$-(2-Mercapto ethyl]-1,2-benzoisothiazole-3,4-diamine-1,1-dioxide

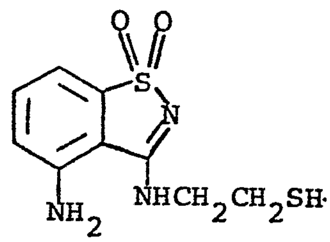

(D.9)

Following the procedures outlined in Example 17, but using cysteamine, 2-methoxyethanol as solvent and at room temperature for 3 days, the title compound was obtained after evaporation to dryness and trituration with methanol.

$^1H$ NMR: $d^6$DMSO δ 2.9 - 3.2 (m, 2H)
3.5 - 3.9 (m, 2H)
2.9 - 3.9 (m, 1H exchanges with $D_2O$)
6.2 - 6.7 (m, 2H exchanges with $D_2O$)
6.9 - 7.2 (m, 2H)
7.2 - 7.5 (m, 1H)

Example 1

Preparation of N-[2-(5-dimethylaminomethylfuran-2-ylmethylthio)ethyl]4-nitro-1,2-benzoisothiazole-3-amine-1,1-dioxide

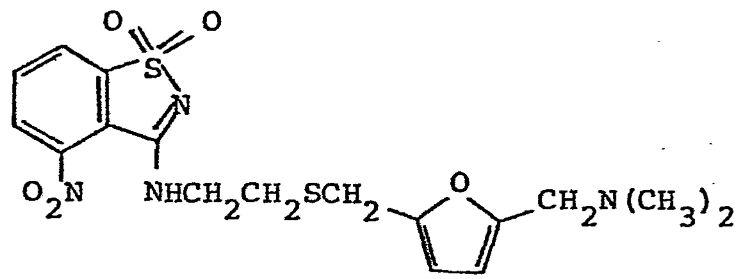

A solution of 4-nitropseudosaccharin chloride (0.43g, 0.00175 mole) in dichloromethane (10ml) was added dropwise to a stirred solution of 2-[(5-dimethylaminomethylfuran-2-yl)methylthio]ethylamine (0.41g, 0.00192 mole) and triethylamine (0.177g, 0.00175 mole) in dichloromethane (10ml) at ambient temperature. The mixture was stirred for 16 hours and was then transferred to a separating funnel and washed with water. The aqueous phase was extracted with dichloromethane (50ml). The combined organic phase was washed with brine (2 x 75ml), dried over magnesium sulphate and evaporated under reduced pressure. The residual crude product was purified, via chromatography on silica gel using 5% methanol chloroform as eluant, to yield the title compound as an orange oil (0.28g) which solidified on standing to yield orange crystals, mpt. 115-6°C.

$^1$H-NMR $CDCl_3$ 79.5 MHz

$\delta$ = 2.27 (s, 6H)
$\delta$ = 2.90 (t, 2H)
$\delta$ = 3.48 (s, 2H)
$\delta$ = 3.65-3.95 (m, 4H)
$\delta$ = 6.1-6.25 (m, 2H)
$\delta$ = 7.9-8.5 (m, 3H)
$\delta$ = 8.75 (br s, 1H)

Mass spectrum $C_{17}H_{20}N_4O_2S_2$ Required 424.0873
Observed 424.0864

Example 2

Preparation of $N^3$-[2-(5-dimethylaminomethylfuran-2-yl methylthio)ethyl]1,2-benzoisothiazole-3,4-diamine-1,1-dioxide

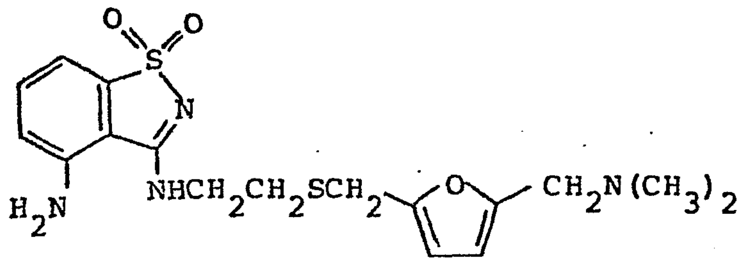

A mixture of electrolytic iron powder (0.080g, N-[2-(5-dimethylaminomethylfuran-2-ylmethylthio)ethyl]1,2-benzothiazole-3-amine 1,1-dioxide (0.170g), glacial acetic acid (0.50ml) and ethanol (10ml) was vigorously stirred under reflux for 2.5 hours. Sodium bicarbonate solution was then added to neutralise the mixture and the mixture was extracted with chloroform (2 x 20ml). The combined organic phase was washed with brine (20ml), dried over magnesium sulphate and evaporated under reduced pressur pressure. The crude residual product was purified by chromatography on alkaline alumina (Brockman type II) using chloroform as eluent to yield the title compound as a yellow gum which solidified on standing to give a white solid, mp 88-90°C, (ethanol/water).

'H-NMR $CDCl_3$ 79.5 MHz

$\delta$ = 2.19 (s, 6H)
$\delta$ = 2.75 (t, 2H)
$\delta$ = 3.3-3.5 (m, 4H including 3.36, s)
$\delta$ = 3.73 (s, 2H)
$\delta$ = 4.2-5.3 (br s, 3H)
$\delta$ = 6.05-6.2 (2d, 2H)
$\delta$ = 6.9-7.2 (dd, 1H)
$\delta$ = 7.25-7.6 (m, 2H)

Mass spectrum $C_{17}H_{22}N_4O_4S$ Required 394.1132
Observed 394.1134

By means of similar procedures to those outlined in Example 1, the following compounds were prepared. The solvents and reaction times were varied as required.

Example 3

N-[2-(5-dimethylaminomethylfuran-2-ylmethylthio)ethyl]-1,2-benzoisothiazole-3-amine-1,1-dioxide

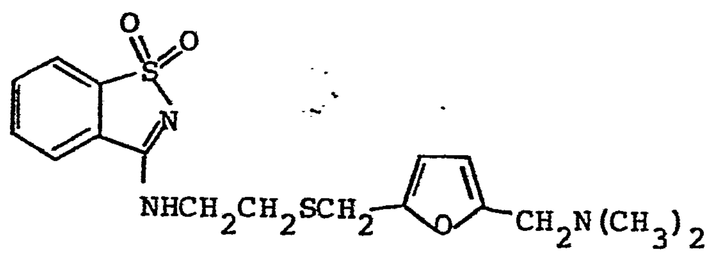

mp 95-8°

'H - NMR $CDCl_3$

$\delta$ = 2.25 (s, 6H)

$\delta$ = 2.85 (t, 2H)

$\delta$ = 3.1-3.95 (m, 7H, 1H exchangable with $D_2O$)

$\delta$ = 6.2 (2d, 2H)

$\delta$ = 7.5-8.0 (m, 5H, 1H exchangable with $D_2O$)

Mass spectrum $C_{17}H_{21}N_3O_3S_2$ Required 379.1024

Observed 379.1002

Example 4

N-[2-(5-dimethylaminomethylfuran-2-ylmethylthio)ethyl]-4-chloro-1,2-benzoisothiazole-3-amine-1,1-dioxide

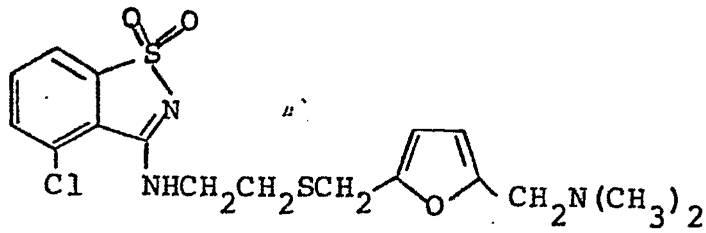

mp 109-10°

'H NMR $CDCl_3$

δ = 2.22 (s, 6H)
δ = 2.87 (t, 3H)
δ = 3.40 (s, 2H)
δ = 3.7-4.0 (m, 4H including 3.77, s)
δ = 6.0-6.3 (2d, 2H)
δ = 7.55-8.0 (m, 4H)

Mass spectrum $C_{17}H_{20}Cl\,N_3O_3S_2$ Required 413.0643
Observed 413.0635

Example 5

N-[2-(5-dimethylaminomethylfuran-2-ylmethylthio)ethyl]5-nitro-1,2-benzoisothiazole-3-amine-1,1-dioxide

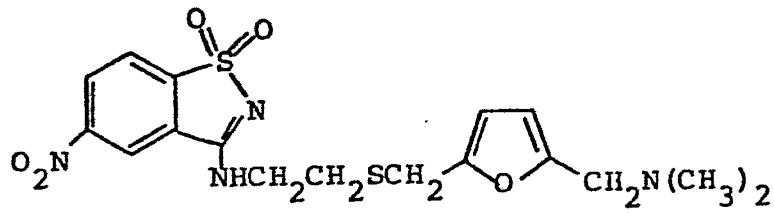

'H NMR $CDCl_3$

δ = 2.38 (s, 6H)
δ = 2.90 (t, 2H)
δ = 3.25-3.80 (m, 6H including 3.58, s; 3.75, s)
δ = 3.85-4.90 (br s, 1H exchangeable with $D_2O$)
δ = 6.05-6.30 (2d, 2H)
δ = 7.95-9.00 (m, 4H; 1H exchangeable with $D_2O$)

Mass spectrum $C_{17}H_{20}N_4O_5S_2$ Required 424.0875
Observed 424.0876

Example 6

N-[2-(5-dimethylaminomethylfuran-2-ylmethylthio)ethyl]-6-nitro-1,2-benzoisothiazole-3-amine-1,1-dioxide

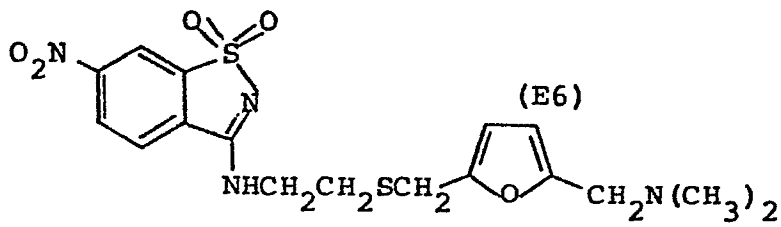

(E6)

1H-NMR $CDCl_3$

δ = 2.23 (s, 6H)
δ = 2.82 (t, 2H)
δ = 3.43 (s, 2H)
δ = 3.58-3.88 (m, 4H)
δ = 6.1 (s, 2H)
δ = 6.4-7.8 (br.s, 1H)
δ = 7.96-8.86 (m, 3H)

Example 7

N-[2-(5-dimethylaminomethylfuran-2-ylmethylthio)ethyl]-7-nitro-1,2-benzoisothiazole-3-amine-1,1-dioxide

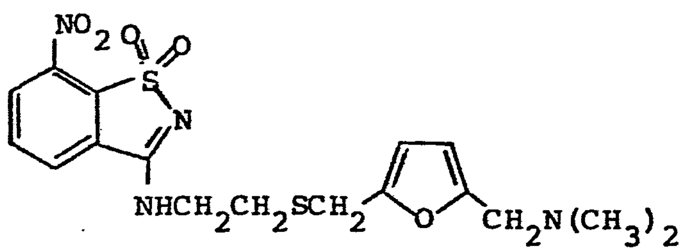

'H NMR $CDCl_3$

δ = 2.37 (s, 6H)
δ = 2.89 (t, 3H)
δ = 3.3-3.8 (m, 7H including 3.58, s and 3.75, s)
δ = 6.24 (s, 2H)
δ = 7.8-8.05 (m, 1H)
δ = 8.35-8.65 (m, 2H)

Example 8

N-[2-(5-dimethylaminomethylfuran-2-ylmethylthio)ethyl]-6-chloro-1,2-benzoisothiazole-3-amine-1,1-dioxide

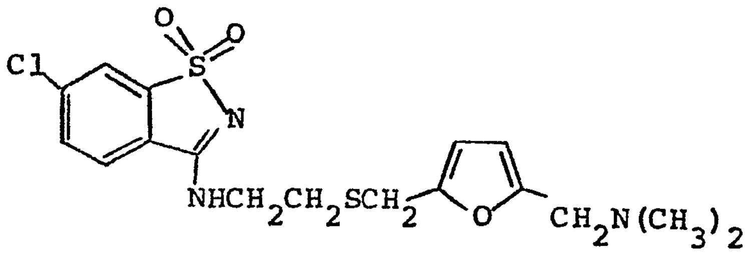

'H NMR $CDCl_3$

δ = 2.37 (s, 6H)
δ = 2.86 (t, 2H)
δ = 3.35-3.85 (m, 6H including 3.59, s and 3.76, s)
δ = 4.25-5.25 (br s, 1H)
δ = 6.1-6.3 (2d, 2H)
δ = 7.5-8.10 (m, 3H)

Mass spectrum $C_{17}H_{20}Cl\ N_3O_3S_2$ Required 413.0635
Observed 413.0635

Example 9

N-[2-(5-Dimethylaminomethylfuran-2-ylmethylthio)ethyl]-6-dimethylsulphamoyl-1,2-benzoisothiazole-3-amine-1,1-dioxide

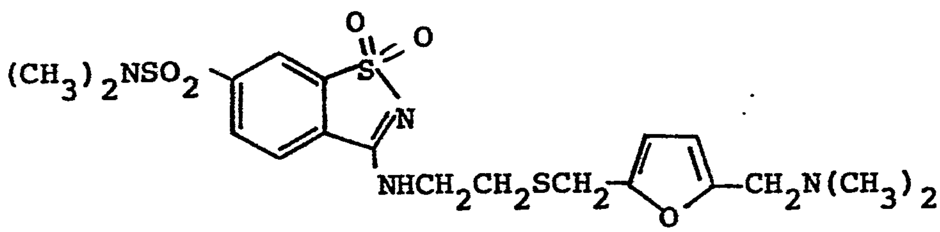

mp 155-8° (dec)

$^1H$ NMR CDCl$_3$

δ = 2.25 (s, 6H)
δ = 2.78 (s, 6H)
δ = 2.87 (t, 2H)
δ = 3.45 (s, 3H)
δ = 3.57 (t, 2H)
δ = 3.76 (s, 2H)
δ = 6.19 (dd, 2H)
δ = 8.06 (m, 2H)
δ = 8.24 (m, 1H)
δ = 7.2-8.5 (brs, 1H)

Mass spectrum $C_{19}H_{26}N_4O_5S_3$ Required 486.1065
Observed 486.1069

Example 10

N-[3-(3-piperid-1-ylmethylphenoxy)propyl]-1,2-benzoisothiazole-3-amine-1,1-dioxide

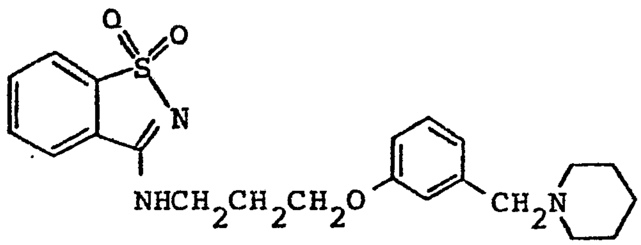

mp 140-2°

'H NMR $CDCl_3$

$\delta$ = 1.3-1.8 (m, 6H)
$\delta$ = 2.0-2.55 (m, 6H)
$\delta$ = 3.48 (s, 2H)
$\delta$ = 3.65-3.95 (br t, 2H)
$\delta$ = 4.13 (t, 2H)
$\delta$ = 6.55-6.7 (m, 5H)
$\delta$ = 7.55-8.0 (m, 4H)

| | | | |
|---|---|---|---|
| Mass spectrum | $C_{22}H_{27}N_3O_3S$ | Required | 413.1773 |
| | | Observed | 413.1769 |

Example 11

N-[3-(3-piperid-1-ylmethylphenoxy)propyl]-4-nitro-1,2-benzoisothiazole-3-amine-1,1-dioxide

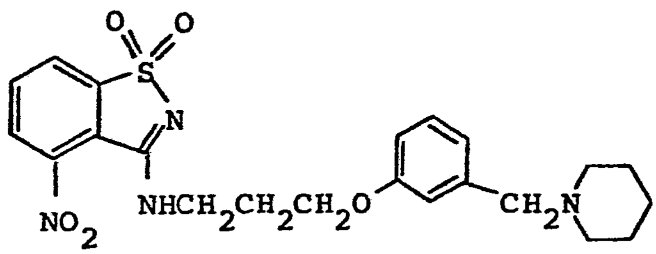

mp 143-5° (dec)

'H NMR $CDCl_3$

δ = 1.25-1.8 (m, 6H)
δ = 2.05-2.6 (m, 6H)
δ = 3.49 (s, 2H)
δ = 3.92 (t, 2H)
δ = 4.06 (t, 2H)
δ = 6.7-7.07 (m, 3H)
δ = 7.07-7.37 (m, 1H)
δ = 7.78-8.1 (m, 2H)
δ = 8.15-8.45 (m, 1H)
δ = 8.3-9.0 (m, 1H)

Mass spectrum $C_{22}H_{26}N_4O_5S$ Required 458.1624
Observed 458.1627

Example 12

N-[3-(3-dimethylaminomethylphenoxy)propyl]-1,2-benzoisothiazole-3-amine-1,1-dioxide

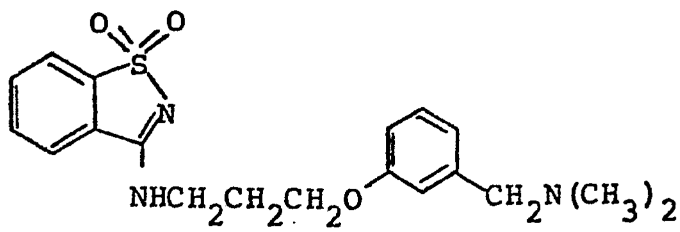

mp 126-9°

'H-NMR $CDCl_3$

δ = 2-2.5 (m, 8H including 2.25, s)
δ = 3.42 (s, 2H)
δ = 3.6-3.95 (br q, 2H; collapses to t with $D_2O$)
δ = 4.14 (t, 2H)
δ = 6.65-7.55 (m, 5H, to 4H with $D_2O$)
δ = 7.55-8.0 (m, 4H)

Mass spectrum $C_{19}H_{23}N_3O_3S$ Required 373.1460
Observed 373.1470

Example 13

N-[2-(2-guanidinothiazol-4-ylmethylthio)ethyl]-4-nitro-1,2-benzoisothiazole-3-amine-1,1-dioxide

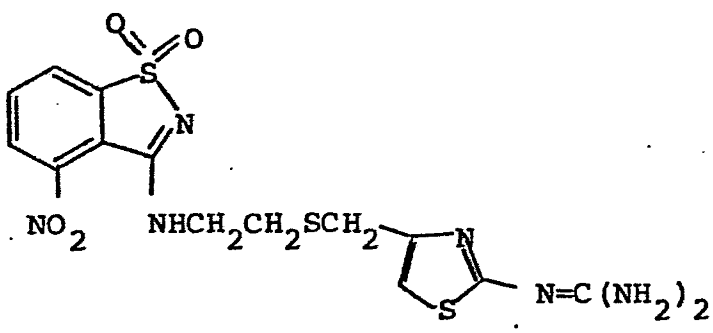

mp 190-1° (dec)

'H-NMR d$^6$DMSO

δ = 2.8 (t, 2H)
δ = 3.5-3.95 (m, 4H including 3.7, s)
δ = 6.6 (s, 1H)
δ = 6.5-7.25 (br s, 4H exchanges with $D_2O$)
δ = 7.85-8.25 (m, 1H)
δ = 8.3-8.55 (m, 2H)
δ = 8.45-9.15 (br s, 1H exchanges with $D_2O$)

| | | | |
|---|---|---|---|
| Mass spectrum | $C_{14}H_{15}N_7O_4S_3$ | Required | 441.0349 |
| | | Observed | 441.0318 |

Example 13a

N-[2-(5-Dimethylaminomethylfuran-2-ylmethylthio)ethyl]-4-iodo-1,2-benzoisothiazole-3-amine-1,1-dioxide

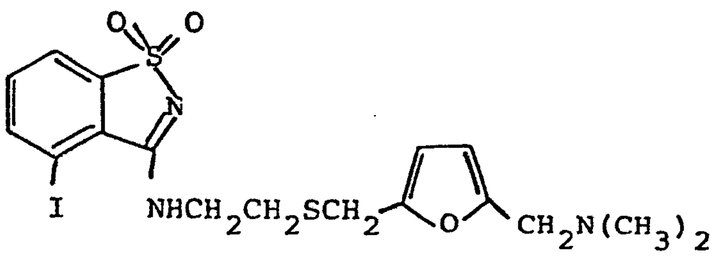

$^1H$-NMR $CDCl_3$

δ = 2.25 (s, 6H)
δ = 2.90 (t, 2H)
δ = 3.42 (s, 2H)
δ = 3.6-4.0 (m, 4H including 3.80, s, 2H)
δ = 6.0-6.25 (2d, 2H)
δ = 7.2-8.25 (m, 4H, 1H exchangeable with $D_2O$)

| | | | |
|---|---|---|---|
| Mass spectrum | $C_{17}H_{20}N_3O_3S_2I$ | Required | 504.9992 |
| | | Observed | 504.9985 |

Example 13b

N-[2-(5-Dimethylaminomethylfuran-2-ylmethylthio)ethyl]-4-cyano-1,2-benzoisothiazole-3-amine-1,1-dioxide

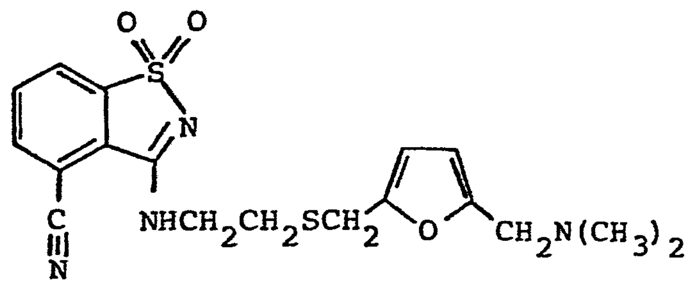

$^1$H-NMR CDCl$_3$

δ = 2.25 (s, 6H)
δ = 2.90 (t, 2H)
δ = 3.44 (s, 2H)
δ = 3.6-4.0 (m, 4H including 3.8, s, 2H)
δ = 6.05-6.25 (2d, 2H)
δ = 7.5-8.25 (m, 3H)
δ = 7.2-8.4 (br m, 1H exchangeable with $D_2O$)

Mass spectrum $C_{18}H_{20}N_4O_3S_2$ Required 404.0977
Observed 404.0954

By means of similar procedures to those outlined in Example 2, the following compounds were prepared. 5N Hydrochloric acid was frequently used in place of glacial acetic acid.

Example 14

$N^3$-[2-(5-dimethylaminomethylfuran-2-ylmethylthio)ethyl]-1,2-benzoisothiazole-3,6-diamine-1,1-dioxide

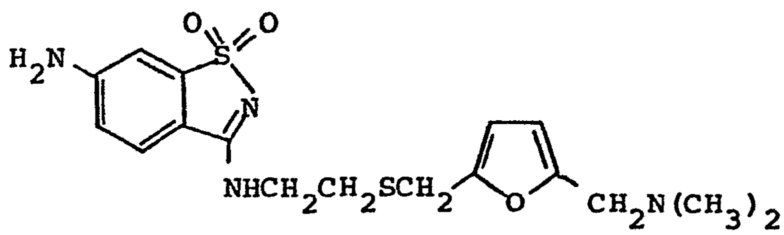

| | | | |
|---|---|---|---|
| Mass spectrum | $C_{17}H_{22}N_4O_3S_2$ | Required | 394.1133 |
| | | Observed | 394.1130 |

Example 15

N[3]-[2-(5-dimethylaminomethylfuran-2-ylmethylthio)ethyl]-1,2-benzoisothiazole-3,7-diamine-1,1-dioxide

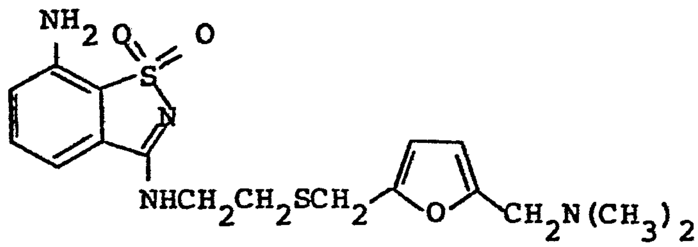

$^1$H-NMR $CDCl_3$

$\delta$ = 2.26 (s, 6H)
$\delta$ = 2.85 (t, 2H)
$\delta$ = 3.25-4.0 (m, 6H including 3.45, s and 3.75, s)
$\delta$ = 4.4-4.9 (br s, 2H exchanges with $D_2O$)
$\delta$ = 6.1-6.3 (2d, 2H)
$\delta$ = 6.75-7.7 (m, 4H, reduces to 3H with $D_2O$)

Example 16

$N^3$-[3-(3-piperid-1-ylmethylphenoxy)propyl]-1,2-benzoisothiazole-3,4-diamine-1,1-dioxide

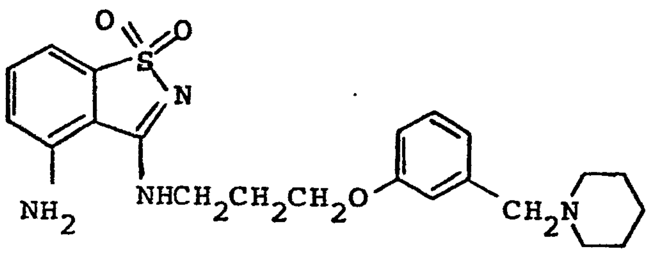

'H-NMR    $CDCl_3$

$\delta$ = 1.26-1.8 (m, 6H)

$\delta$ = 1.8-2.2 (m, 2H)

$\delta$ = 2.2-2.55 (m, 4H)

$\delta$ = 3.25-3.6 (m, 4H including 3.45, s)

$\delta$ = 4.06 (t, 2H)

$\delta$ = 4.2-4.7 (br s, 2H exchanges with $D_2O$)

$\delta$ = 6.65-7.6 (m, 7H)

$\delta$ = 7.35-8.2 (br s, 1H exchanges with $D_2O$)

Example 17

N-[2-(5-dimethylaminomethylfuran-2-ylmethylthio)ethyl]-5,6-dimethoxy-1,2-benzoisothiazole-3-amine-1,1-dioxide

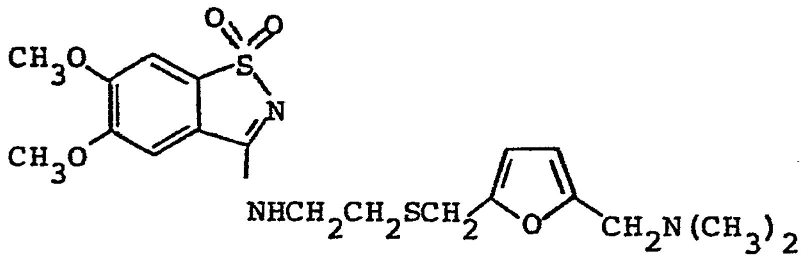

A toluene (50ml) solution of 5,6-dimethoxy-3-ethoxy-1,2-benzoisothiazole-1,1-dioxide (D5) (0.13g) was heated under reflux with 2-[(5-dimethylaminomethylfuran-2-yl)methylthio] ethylamine (0.2g) for 18h. Evaporation of the solvent and purification of the residue by column chromatography on silica gel using 5% methanolic chloroform as eluant afforded the title compound (0.1g).

'H-NMR $CDCl_3$

δ = 2.25 (s, 6H)
δ = 2.65-3.0 (t, 2H)
δ = 3.3-4.3 (m, 12H including 3.43, s, 3.71, s and 3.96, s)
δ = 6.0-6.25 (2d, 2H)
δ = 7.27 (s, 1H)
δ = 7.43 (s, 1H)
δ = 7.75-8.2 (br s, 1H)

Mass spectrum $C_{19}H_{25}N_3O_5S_2$ Required 439.1236
Observed 439.1235

Example 17b

$N^3$-[2-(5-dimethylaminomethylfuran-2-ylmethylthio)ethyl]-1,2-benzoisothiazole-3,4-diamine-1,1-dioxide

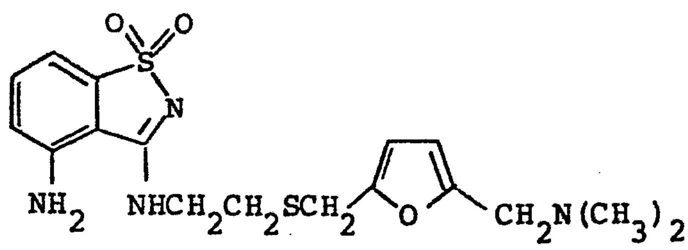

Following the procedures outlined in Example 17, but using methanol as the solvent and heating under reflux for 24h, 4-amino-3-ethoxy-1,2-benzoisothiazole-1,1-dioxide (D3a) was converted into the title compound

mp 88-91° (Ethanol/water)
112-4° (Ethyl acetate/petrol).

Example 18

N³-[2-(5-dimethylaminomethylfuran-2-ylmethylthio)ethyl]-6-acetamido-1,2-benzoisothiazole-3-amine-1,1-dioxide

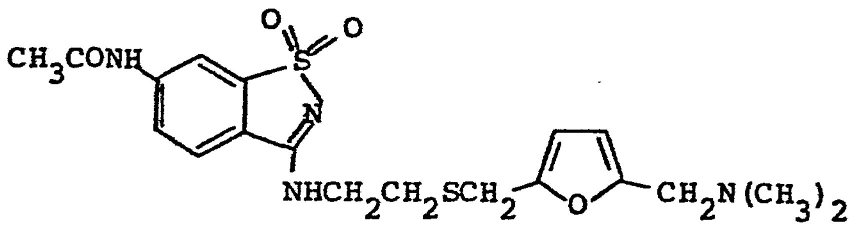

Following the procedures outlined in Example 17, but now using 2-methoxyethanol as solvent and heating under reflux for 3h, 6-acetamido-3-ethoxy-1,2-benzoisothiazole-1,1-dioxide (D.4) (0.2g) was converted into the title compound (0.19g)

mp 151-3°

¹H-NMR $CDCl_3 + CD_3OD$

$\delta$ = 2.0 (s, 3H)
$\delta$ = 2.28 (s, 6H)
$\delta$ = 2.81 (t, 2H)
$\delta$ = 3.45 (s, 2H)
$\delta$ = 3.60 (t, 2H)
$\delta$ = 3.75 (s, 2H)
$\delta$ = 6.1-6.25 (2d, 2H)
$\delta$ = 7.7-7.9 (m, 2H)
$\delta$ = 8.20 (d,d, 1H)

Mass spectrum $C_{19}H_{24}N_4O_4S_2$ Required 436.1243
Observed 436.1239

Example 19

$N^3$-[3-(3-piperid-1-ylmethylphenoxy)propyl]-1,2-benzoisothiazole-3,6-diamine-1,1-dioxide

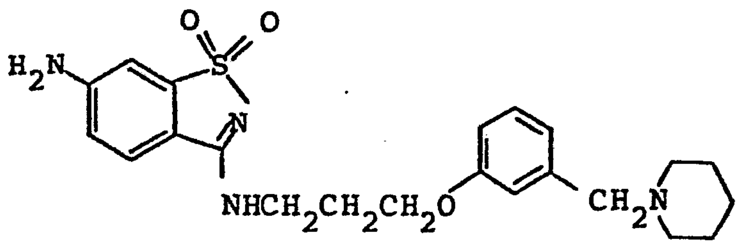

Following the procedures outlined in Example 18 but heating under reflux for 18h, 6-amino-3-ethoxy-1,2-benzoisothiazole-1,1-dioxide (D3b) (1.0g) was converted into the title compound (1.2g)

mp 185-7°

$^1$H-NMR $CDCl_3 + CD_3OD$

δ = 1.3-1.8 (m, 6H)
δ = 2.16 (quin. 2H)
δ = 2.3-2.6 (m, 4H)
δ = 3.51 (s, 2H)
δ = 3.70 (t, 2H)
δ = 4.09 (t, 2H)
δ = 6.6-7.6 (m, 8H)

| | | | |
|---|---|---|---|
| Mass spectrum | $C_{22}H_{28}N_4O_3S$ | Required | 428.1882 |
| | | Observed | 428.1886 |

Example 20

N³-[2-(2-guanidinothiazole-4-ylmethylthio)ethyl]-1,2-benzoisothiazole-3,4-diamine-1,1-dioxide

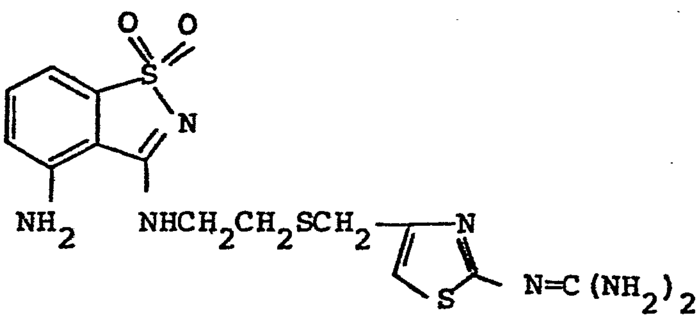

Following the procedures outlined in Example 17, but using methanol as solvent and heating under reflux for 60h, 4-amino-3-ethoxy-1,2-benzoisothiazole-1,1-dioxide (D3a) (0.5g) was converted into the title compound (0.06g)

mp 200-2° (dec)

$^1$H-NMR $d^6$-DMSO

δ = 2.76 (t, 2H)
δ = 3.5-3.8 (m, 4H including 3.65, s)
δ = 6.15-6.43 (br s, 2H; exchanges with $D_2O$)
δ = 6.56 (s, 1H)
δ = 6.65-6.90 (br s, 4H; exchanges with $D_2O$)
δ = 6.9-7.2 (m, 2H)
δ = 7.32-7.6 (m, 1H)
δ = 7.7-8.6 (br s, 1H, exchanges with $D_2O$)

Example 21

N-[2-(5-dimethylaminomethylfuran-2-ylmethylthio)ethyl]-4-methyl-1,2-benzoisothiazole-3-amine-1,1-dioxide

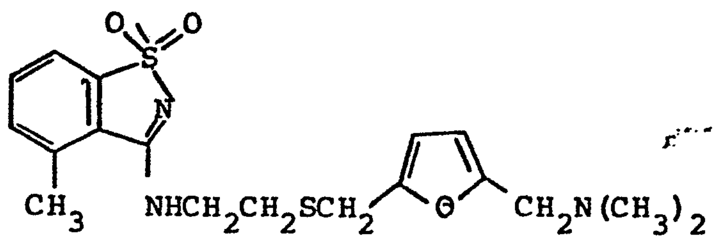

Following the procedures outlined in Example 18, 3-ethoxy-4-methyl-1,2-benzoisothiazole-1,1-dioxide (D2d) was converted into the title compound. mp 90-92°

'H-NMR $CDCl_3$

δ = 2.18 (s, 6H)

δ = 2.72 (s, 3H)

δ = 2.85 (t, 2H)

δ = 3.35 (s, 2H)

δ = 3.55-3.83 (m, 4H, including s, 3.75)

δ = 6.05-6.2 (2d, 2H)

δ = 6.6-7 (br s, 1H)

δ = 7.3-7.85 (m, 3H)

Following the procedures outlined in Example 18, the following Examples were prepared:

Example 21b

N-[3-(3-Piperid-1-ylmethylphenoxy)propyl]-6-dimethylsulphamoyl-1,2-benzoisothiazole-3-amine-1,1-dioxide.

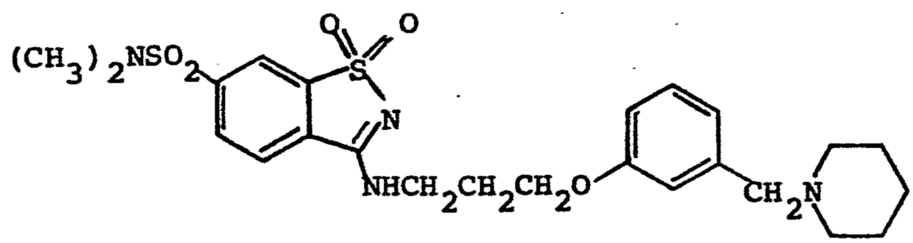

| | | | |
|---|---|---|---|
| $^1H$ NMR | $CDCl_3 + D_2O$ | | |
| | δ = | 1.25-1.8 | (m, 6H) |
| | δ = | 2.0 -2.65 | (m, 6H) |
| | δ = | 2.8 | (s, 6H) |
| | δ = | 3.50 | (s, 2H) |
| | δ = | 3.83 | (t, 2H) |
| | δ = | 4.16 | (t, 2H) |
| | δ = | 6.60-7.4 | (m, 4H) |
| | δ = | 8.05 | (m, 2H) |
| | δ = | 8.24 | (m, 1H) |

Example 21c

N-[2-(2-Guanidinothiazole-4-ylmethylthio)ethyl]-6-dimethylsulphamoyl-1,2-benzoisothiazole-3-amine-1,1-dioxide

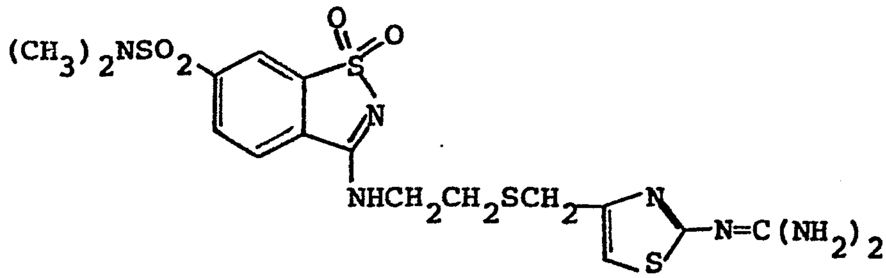

$^1$H NMR $d^6$ DMSO

| | |
|---|---|
| $\delta$ = 2.7 | (s, 6H) |
| $\delta$ = 2.8 | (t, 2H) |
| $\delta$ = 3.16 | (s, 2H) |
| $\delta$ = 3.7 | (m) |
| $\delta$ = 6.56 | (s, 1H) |
| $\delta$ = 6.6-7.2 | (brs, 4H exchanges with $D_2O$) |
| $\delta$ = 8.1-8.65 | (m, 3H) |
| $\delta$ = 9.6-10.1 | (brs, 1H exchanges with $D_2O$) |

Mass spectrum $C_{16}H_{21}N_7O_4S_4$ Required 503.0539
Observed 503.0544

Example 21d

N-[2-(5-Dimethylaminomethylfuran-2-ylmethylthio)ethyl]-6-methylsulphamoyl-1,2-benzoisothiazole-3-amine-1,1-dioxide

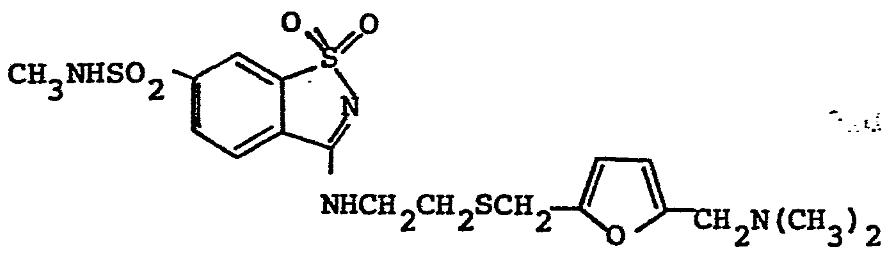

mp 200-202°C (dec)

$^1$H NMR CDCl$_3$ + CD$_3$OD

δ = 2.29 (s, 6H)
δ = 2.65 (s, 3H)
δ = 2.85 (t, 2H)
δ = 4.50 (s, 2H)
δ = 3.70 (t, 2H)
δ = 3.80 (s, 2H)
δ = 6.22 (2d, 2H)
δ = 8.17 (m, 2H)
δ = 8.33 (m, 1H)

Example 21e

N-[3-(3-Piperid-1-ylmethylphenoxy)propyl]-6-methanesulphonylamino-1,2-benzoisothiazole-3-amine-1,1-dioxide

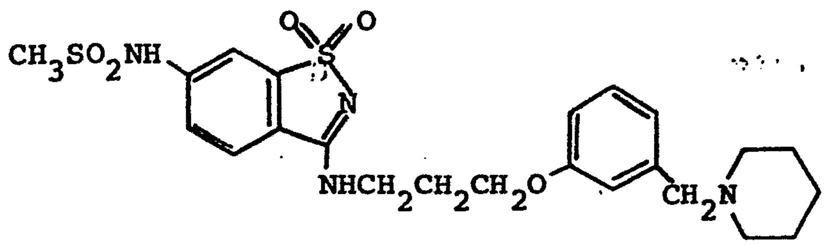

$^1H$-NMR $CDCl_3$ + $d^6$ DMSO

δ = 1.25-1.75 (m, 6H)
δ = 2.0 -2.6 (m, 6H)
δ = 3.05 (s, 3H)
δ = 3.50 (s, 2H)
δ = 3.72 (brq, 2H)
δ = 4.08 (t, 2H)
δ = 6.6-7.35 (m, 4H)
δ = 7.54 (d,d, 1H)
δ = 7.70 (d, 1H)
δ = 8.07 (d, 1H)
δ = 9.12 (brt, 1H)

Mass spectrum $C_{23}H_{30}N_4O_5S_2$ Required 506.16575
Observed 506.16560

Example 21f

N-[2-(2-Guanidinothiazole-4-ylmethylthio)ethyl]-6-acetamido-1,2-benzoisothiazole-3-amine-1,1-dioxide

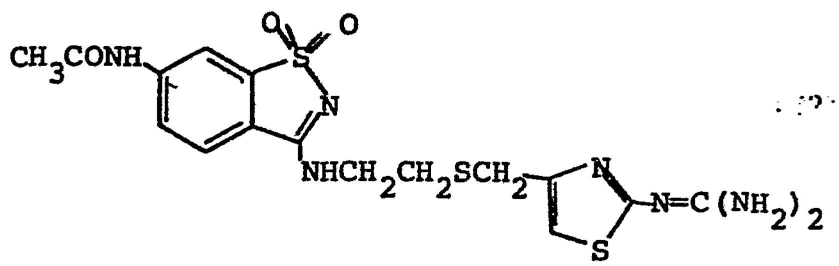

$^1$H NMR $d^6$ DMSO

| | |
|---|---|
| δ = 2.15 | (s, 3H) |
| δ = 2.79 | (t, 2H) |
| δ = 3.3-3.75 | (m, 2H) |
| δ = 3.70 | (s, 2H) |
| δ = 6.6 | (s, 1H) |
| δ = 6.95 | (brs, 4H) |
| δ = 7.75-8.30 | (m, 3H) |
| δ = 9.50 | (brt, 1H) |
| δ = 10.65 | (brs, 1H) |

Example 21g

N-[3-(3-Piperid-1-ylmethylphenoxy)propyl]-6-iodo-1,2-benzoisothiazole-3-amine-1,1-dioxide

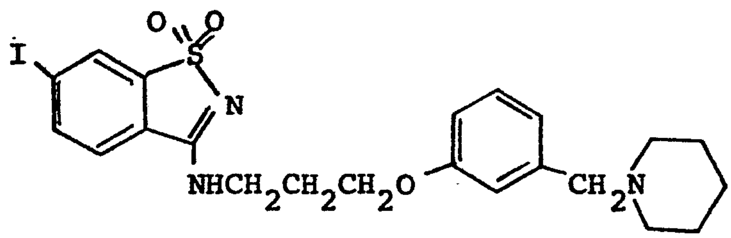

mp 169-7°C

$^{1}H$ NMR CDCl$_3$

$\delta$ = 1.55 (m, 6H)
$\delta$ = 2.20 (t, 2H)
$\delta$ = 2.30-2.70 (m, 4H)
$\delta$ = 3.50 (s, 2H)
$\delta$ = 3.80 (t, 2H)
$\delta$ = 4.15 (t, 2H)
$\delta$ = 6.7-7.7 (m, 6H)
$\delta$ = 8.03 (d,d, 1H)
$\delta$ = 8.24 (d, 1H)

Mass spectrum $C_{22}H_{26}N_3O_3SI$ Required 534.0742
Observed 539.0744

Example 21h

N-[2-(2-Guanidinothiazole-4-ylmethylthio)ethyl]-1,2-benzoisothiazole-3,6-diamine-1,1-dioxide

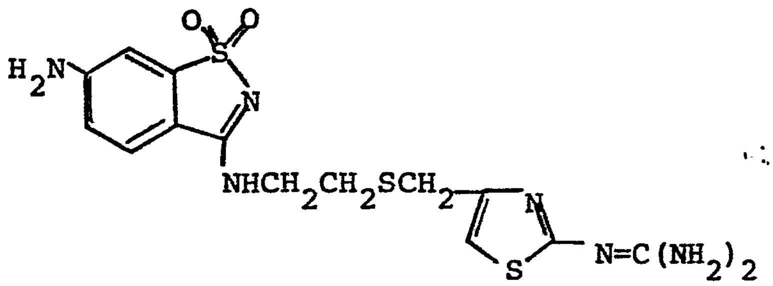

mp 217-222°C (dec)

$^1H$ NMR : $d^6$ DMSO

| | |
|---|---|
| δ = 2.72 | (t, 2H) |
| δ = 3.50 | (s, 2H) |
| δ = 3.65 | (t, 2H including s, 1½H ½ mole MeOH) |
| δ = 6.40 | (s, 2H) |
| δ = 6.55 | (s, 1H) |
| δ = 6.6-7.0 | (m, 6H) |
| δ = 7.74 | (d, 1H) |
| δ = 8.85-9.20 | (brt, 1H) |

Mass spectrum $C_{14}H_{17}N_7O_2S_3$ Required 411.0607
Observed 411.0613

Example 21i

N-[3-(3-Piperid-1-ylmethylphenoxy)propyl]-6-propionamido-1,2-benzoisothiazole-3-amine-1,1-dioxide

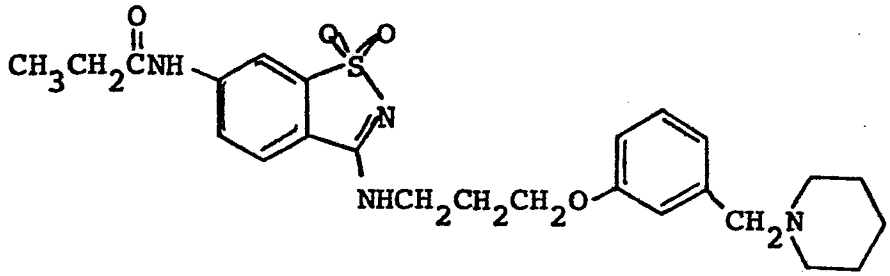

mp 128-30°C

$^1$H NMR CDCl$_3$ + CD$_3$OD

δ = 1.22 (t, 3H)
δ = 1.4-1.67 (m, 6H)
δ = 2.23 (quin. 2H)
δ = 2.35-2.52 (m, 6H)
δ = 3.47 (s, 2H)
δ = 3.75 (t, 2H)
δ = 4.10 (t, 2H)
δ = 6.8-7.4 (m, 3H)
δ = 7.22 (t, 1H)
δ = 7.87 (d, 1H)
δ = 8.02 (d,d, 1H)
δ = 8.10 (d, 1H)

Mass spectrum $C_{25}H_{32}N_4O_4S$ Required 484.2144
Observed 484.2134

Example 21j

N-[3-(3-Piperid-1-ylmethylphenoxy)propyl]-6-formamido-1,2-benzoisothiazole-3-amine-1,1-dioxide

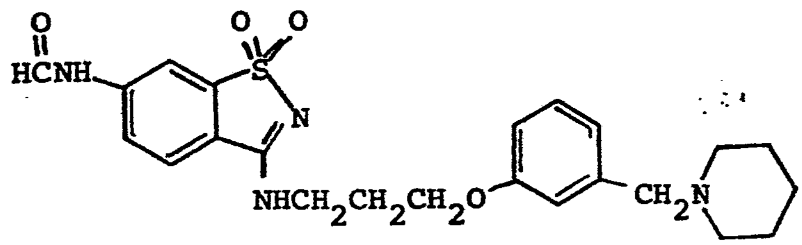

$^1$H NMR CDCl$_3$

δ = 1.0-1.85 (m, 6H)
δ = 1.85-2.75 (m, 6H)
δ = 3.2-4.3 (m, 6H including 3.45, s, 2H)
δ = 6.50-8.50 (m, 8H)

Mass spectrum $C_{23}H_{28}N_4O_4S$ Required 456.1831
Observed 456.1822

Example 22

N³-[2-(5-dimethylaminomethylfuran-2-ylmethylthio)ethyl]-5-amino-1,2-benzoisothiazole-3-amine-1,1-dioxide

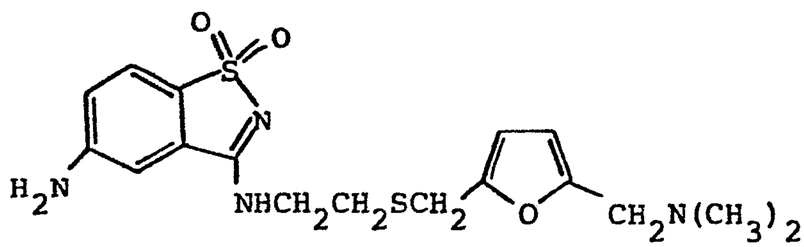

Following the procedures outlined in Example 18, 5-amino-3-ethoxy-1,2-benzoisothiazole-1,1-dioxide (D3c) (0.45g) was converted to the title compound (0.11g)

mp 200-2°

$^1$H-NMR $CDCl_3$

δ = 2.25 (s, 6H)
δ = 2.75-3.0 (br t, 2H)
δ = 3.3-3.8 (m, 8H including 3.44, s, 2H; 3.50, s, 2H, exchangeable with $D_2O$ and 3.75, s, 2H)
δ = 6.05-6.25 (2d, 2H)
δ = 7.75-8.6 (m, 4H, 1H exchangeable with $D_2O$)

Example 23

N³-[4-(3-piperid-1-ylmethylphenoxy)butyl]-1,2-benzoisothiazole-3,4-diamine-1,1-dioxide

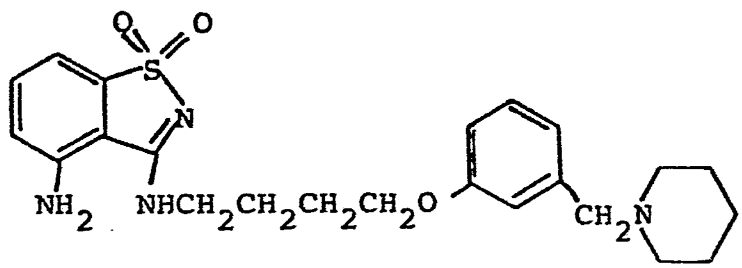

Following the procedures outlined in Example 17, but using methanol as solvent at room temperature for 16h, 4-amino-3-ethoxy-1,2-benzoisothiazole-1,1-dioxide (D3a) (0.3g) was converted into the title compound (0.42g).

mp 135-135.5°

$^1$H-NMR $CDCl_3$

δ = 1.25-2.0 (m, 10H)
δ = 2.3-2.65 (m, 4H)
δ = 3.0-3.35 (br t, 2H)
δ = 3.52 (s, 2H)
δ = 3.8-4.1 (br t, 2H)
δ = 4.25-4.85 (br s, 2H; exchanges with $D_2O$)
δ = 6.65-7.75 (m, 8H; 1H exchanges with $D_2O$)

| | | | |
|---|---|---|---|
| Mass spectrum | $C_{23}H_{30}N_4O_3S$ | Required | 442.2039 |
| | | Observed | 442.2045 |

Example 24

N'-[3-(3-piperid-1-ylmethylphenoxy)propyl]-6-acetamido-1,2-benzoisothiazole-3-amine-1,1-dioxide

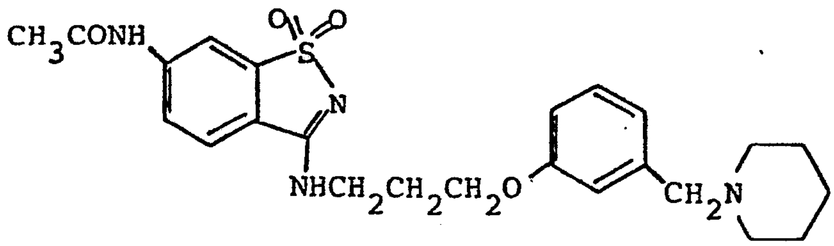

Following the procedures outlined in Example 18, 6-acetamido-3-ethoxy-1,2-benzoisothiazole-1,1-dioxide (D4a) (0.18g) was converted into the title compound (0.15g).

mp 193-5°

$^1$H-NMR  $CDCl_3$ + $d_6$ DMSO

δ = 1.2-1.8 (m, 6H)
δ = 1.9-2.6 (m, 9H including 2.16, s, 3H)
δ = 3.44 (s, 2H)
δ = 3.3-3.85 (m, 2H)
δ = 3.9-4.2 (t, 2H)
δ = 6.6-7.3 (m, 4H)
δ = 7.7-8.3 (m, 3H)
δ = 8.9-9.2 (brt, 1H exchangeable with $D_2O$)
δ = 10.2-10.5 (brs, 1H exchangeable with $D_2O$)

Example 25

N³-[2-(5-Dimethylaminomethylfuran-2-ylmethylthio)ethyl]-6-methylcarbamoylamino-1,2-benzoisothiazole-3-amine-1,1-dioxide

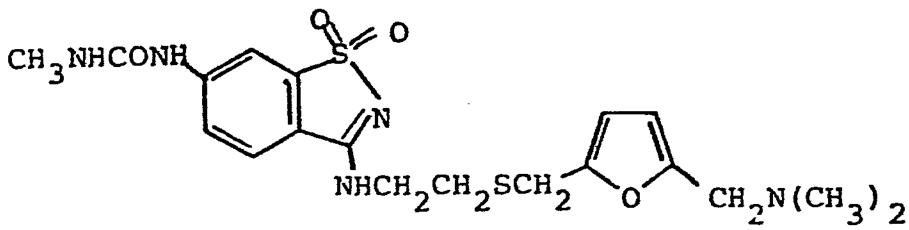

Following the procedures outlined in Example 18, 6-methylcarbamoylamino-3-ethoxy-1,2-benzoisothiazole-1,1-dioxide (D4b) (0.23g) was converted to the title compound (0.1g)

$^1$H-NMR $CDCl_3$ + $CD_3OD$

δ = 2.29 (s, 6H)
δ = 2.7-2.95 (m, 5H including 2.83, s, 3H)
δ = 3.45-3.75 (m, 6H including 3.50, s, 2H and 3.78, s, 2H)
δ = 6.1-6.25 (2d, 2H)
δ = 7.6-8.05 (m, 3H)

Example 26

$N^3$-[2-(5-Dimethylaminomethylfuran-2-ylmethylthio)ethyl]-4-acetamido-1,2-benzoisothiazole-3-amine-1,1-dioxide

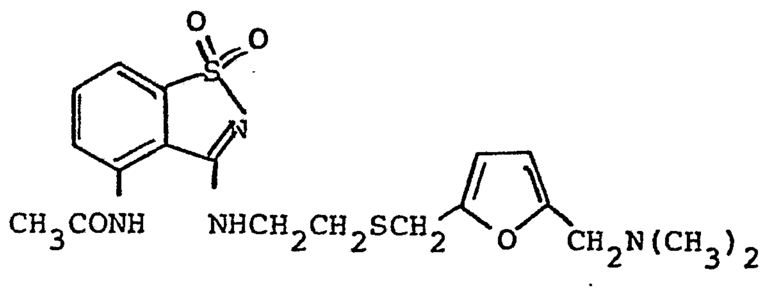

Following the procedures outlined in Description 4, $N^3$-[2-(5-dimethylaminomethylfuran-2-ylmethylthio)ethyl]-1,2-benzoisothiazole-3,4-diamine-1,1-dioxide (0.27g) was converted to the title compound (0.22g)

$^1$H-NMR CDCl$_3$ + D$_2$O

δ = 2.08 (s, 6H)
δ = 2.36 (s, 3H)
δ = 2.82 (t, 2H)
δ = 3.3-3.66 (m, 4H including 3.41, s, 2H)
δ = 3.78 (s, 2H)
δ = 6.18 (s, 2H)
δ = 7.55-7.9 (m, 3H)

Example 27

N³-[3-(3-Piperid-1-ylmethylphenoxy)propyl]-1,2-benzoisothiazole-3-amine-1-oxide

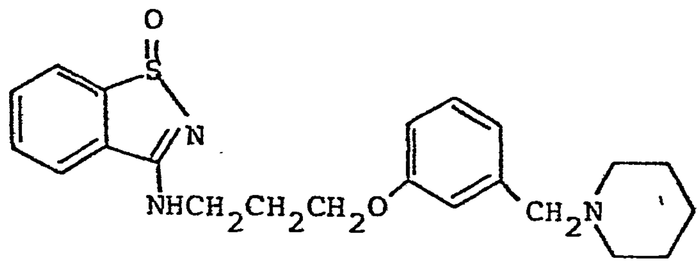

A solution of N-[3-(3-Piperid-1-ylmethylphenoxy)propyl]-1,2-benzoisothiazole-3-amine (D7) (0.2g) in methanol (20ml) and 5N hydrochloric acid (5ml) was stirred at room temperature with m-chloroperbenzoic acid (0.1g) for 30 min. Concentration, basification with potassium carbonate and extraction with dichloromethane afforded, on evaporation to dryness, the title compound (0.15g).

$^1$H-NMR $CDCl_3$

$\delta$ = 1.2-1.8 (m, 6H)
$\delta$ = 1.95-2.6 (m, 6H)
$\delta$ = 3.43 (s, 2H)
$\delta$ = 3.6-3.9 (br d,t 2H)
$\delta$ = 4.09 (t, 2H)
$\delta$ = 6.7-8.1 (m, 9H, 1H exchanges with $D_2O$)

EXAMPLE 28

N-[3-(3-{1-Piperidylmethyl}phenoxy)propyl]-6-N-methyl-sulphamoyl-1,2-benzoisothiazole-3-amine-1,1-dioxide

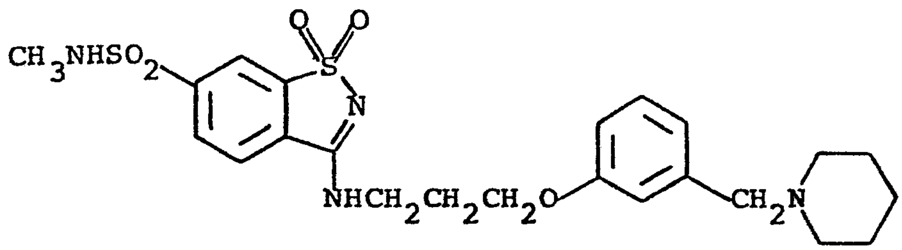

A solution of 3-methylthio-6-N-methylsulphamoyl-1,2-benzoisothiazole-1,1-dioxide(0.53g) and 3-(3-{piperidylmethyl}phenoxy)propylamine (0.43g) in dimethoxyethane (15 ml) and methanol (10 ml) was stirred at room temperature for 24 hrs. On evaporation, the residue was purified by column chromatography (silica, 5% MeOH/$CHCl_3$) to give the title compound as a foam (0.5 g).

| | | |
|---|---|---|
| NMR ($CDCl_3$) | δ = 8.4 - 7.9 | (m., 3H) |
| | 7.85- 6.65 | (m., 4H) |
| | 6.5 - 5.0 | (br s., 2H exchanges with $D_2O$) |
| | 4.09 | (t, 2H) |
| | 3.75 | (t, 2H) |
| | 3.57 | (s, 2H) |
| | 2.85- 2.0 | (br m., 9H) |
| | 2.0 - 1.2 | (br m., 6H) |

| | | |
|---|---|---|
| Mass Spectrum | $C_{23}H_{30}N_4O_5S_2$ | Required 506.1658 |
| | | Observed 506.1670 |

FOLLOWING THE PROCEDURE OUTLINED IN EXAMPLE 28, THE FOLLOWING COMPOUNDS WERE PREPARED:

EXAMPLE 29

N-[2-(5-Dimethylaminomethylfuran-2-ylmethylthio)ethyl]-6-sulphamoyl-1,2-benzoisothiazole-3-amine-1,1-dioxide

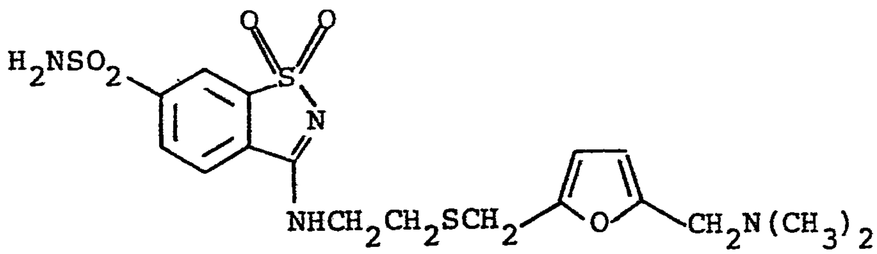

EXAMPLE 30

N-[3-(3-{1-Piperidylmethyl}phenoxy)propyl]-6-sulphamoyl-1,2,-benzoisothiazole-3-amine-1,1-dioxide

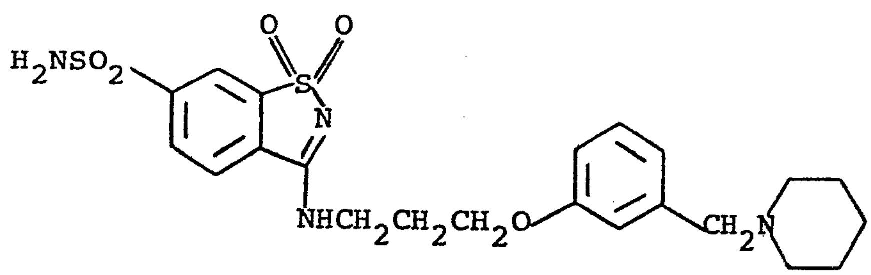

EXAMPLE 31

N-[2-(2-Guanidinothiazole-4-ylmethylthio)ethyl]-6-sulphamoyl-1,2-benzoisothiazole-3-amine-1,1-dioxide

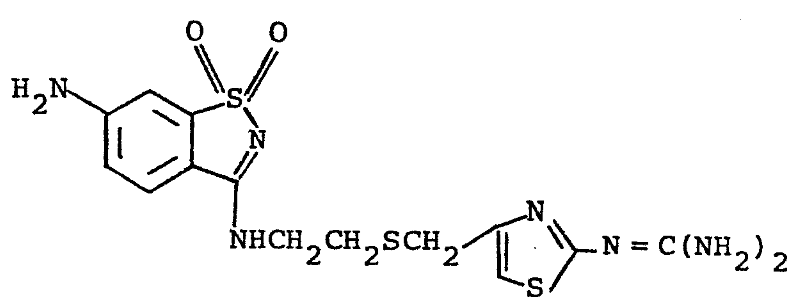

EXAMPLE 33

N-[2-(5-Dimethylaminomethylfuran-2-ylmethylthio)ethyl]-7-dimethylsulphamoyl-1,2,4-benzothiadiazine-3-amine-1,1,dioxide

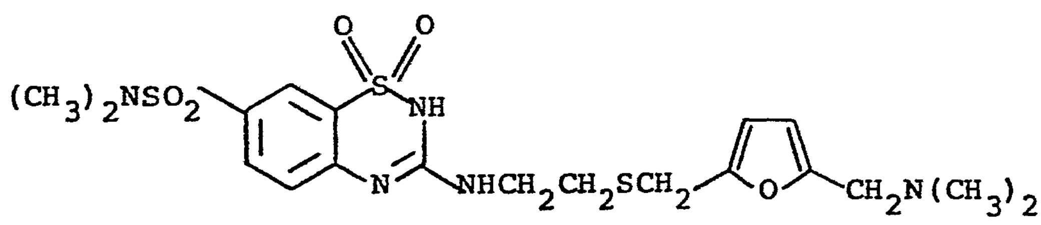

A stirred solution of 2-(5-dimethylaminomethylfuran-2-ylmethylthio)ethylamine (0.33g), 3-chloro-7-dimethylsulphamoyl-1,2,4-benzothiadiazine-1,1-dioxide (0.5g) and triethylamine (0.21ml) in isopropanol (50ml) was heated under reflux for 3 hrs. On evaporation to dryness, aqueous sodium bicarbonate solution was added and the product extracted with chloroform (2 x 100ml). Purification by column chromatography (silica, 10% MeOH/$CHCl_3$) afforded the title compound as a foam (0.3g).

$^1$H-NMR ($CDCl_3$) δ = 2.38 (s, 6H)
2.71 (br s, 8H)
3.3 - 3.8 (m, 6H)
6.28 (2d, 2H)
7.15 (d, 1H)
7.80 (dd, 1H)
8.23 (d, 1H)

EXAMPLE 34

N-[3-(3-Piperid-1-ylmethylphenoxy)propylamino]-7-dimethylsulphamoyl-1,2,4-benzothiadiazine-3-amine-1,1-dioxide

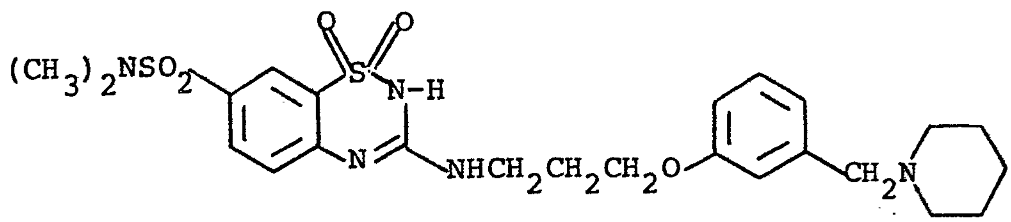

Following the procedure outlined in Example 33, the title compound was obtained as a foam.

| | | | |
|---|---|---|---|
| $^1H$ NMR $CDCl_3$ (270 MHz) δ = | 1.4 - 1.55 | (m, 2H) | |
| | 1.55- 1.72 | (m, 4H) | |
| | 1.95- 2.10 | (m, 2H) | |
| | 2.5 - 2.8 | (m, 10H including 2.68, s, 6H) | |
| | 3.56 | (t, 2H) | |
| | 3.63 | (s, 2H) | |
| | 4.02 | (t, 2H) | |
| | 6.75- 6.9 | (m, 2H) | |
| | 7.0 | (s, 1H) | |
| | 7.14- 7.3 | (m, 2H) | |
| | 7.73 | (dd, 1H) | |
| | 8.17 | (d, 1H) | |

Mass spectrum $C_{24}H_{33}N_5O_5S_2$ Required 535.1923
Observed 535.1919

EXAMPLE 35

N-[2-(2-Guanidinothiazole-4-ylmethylthio)ethyl]-7-dimethylsulphamoyl-1,2,4-benzothiadiazine-3-amine-1,1-dioxide

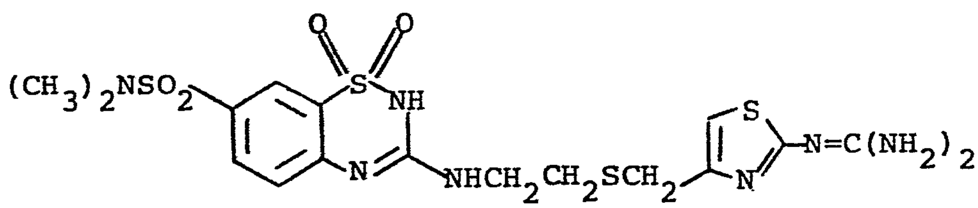

Following the procedure outlined in Example 33, the title compound was obtained as a foam.

EXAMPLE 36

N-[3-(3-Piperid-1-ylmethylphenoxy)propylamino]-7-bromo-1,2,4-benzothiadiazine-3-amine-1,1-dioxide monohydrate

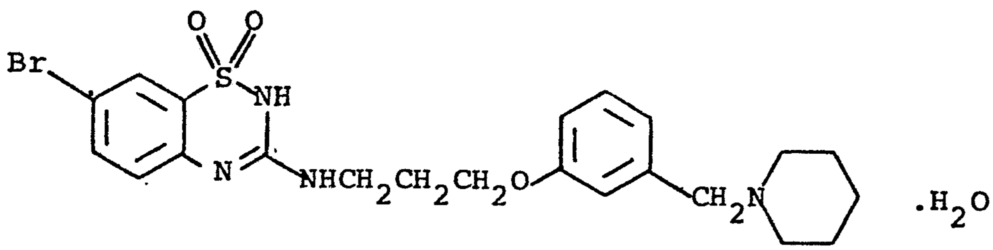

Following the procedure outlined in Example 33, the title compound was obtained as a foam.

$^1$H NMR $CDCl_3$ δ 1.25 - 1.8 (m, 6H)
1.8 - 2.2 (m, 2H)
2.3 - 2.7 (m, 4H)
3.3 - 3.7 (m, 4H)
4.0 (t, 2H)
6.1 - 7.3 (m, 9H including 4H exchangeable with $D_2O$)
7.5 (dd, 1H)
7.88 (d, 1H)

EXAMPLE 32

$N^3$-[2-(5-Dimethylaminomethylfuran-2-ylmethylthio)ethyl]-1,2-benzoisothiazole-3,4-diamine-1,1-dioxide

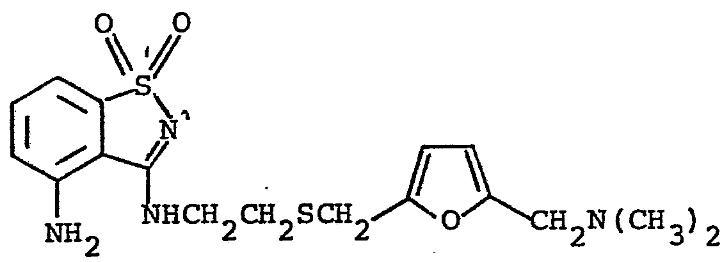

To a stirred solution of $N^3$-[2-mercapto ethyl]-1,2-benzoisothiazole-3,4-diamine-1,1-dioxide (D.9, 0.11g) in concentrated hydrochloric acid (0.4ml) under nitrogen at -10°C was added 5-dimethylaminomethyl-2-furanmethanol (0.08g). After stirring at room temperature overnight, the reaction mixture was worked-up as described in Example 2 to afford the title compound.

Following the procedure outlined in Example 33 , the following further compounds were prepared.

Example 37

N-[2-({5-Dimethylaminomethylfuran-2-yl}methylthio)ethyl]-2-methyl-1,2,4-benzothiadiazine-3-amine-1,1-dioxide

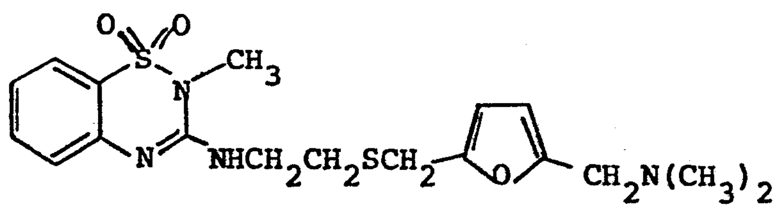

| | | |
|---|---|---|
| NMR (δ, $CDCl_3$) | 7.85-7.0 | (m, 4H) |
| | 6.14 | (s, 2H) |
| | 5.6-5.3 | (m, 1H) |
| | 4.8-3.25 | (m, 9H including 3.75 s, 2H; 3.40, s, 2H and 3.35, s, 3H) |
| | 2.81 | (t, 2H) |
| | 2.25 | (s, 6H) |

Example 37b

N-[2-({5-Dimethylaminomethylfuran-2-yl}methylthio)ethylamino]-4-methyl-1,2,4-benzothiadiazine-3-amine-1,1-dioxide

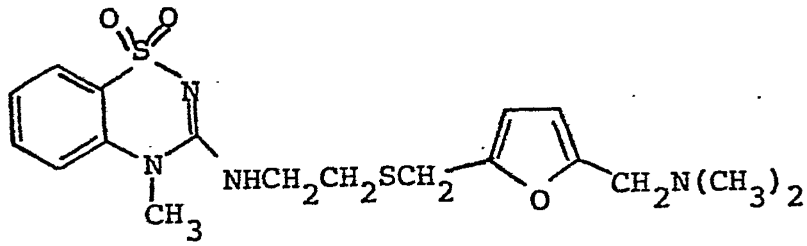

$^1H$ NMR CDCl$_3$

δ = 2.30 (s, 6H)
δ = 2.75 (t, 2H)
δ = 3.25-3.70 (m, 9H including 3.51, s, 5H; and 3.69, s, 2H)
δ = 6.17 (brs, 2H)
δ = 6.45 (brt, 1H)
δ = 7.0-8.0 (m, 4H)

Example 37c

N-[3-(3-Piperid-1-ylmethylphenoxy)propylamino]-4-methyl-1,2,4-benzothiadiazine-3-amino-1,1-dioxide

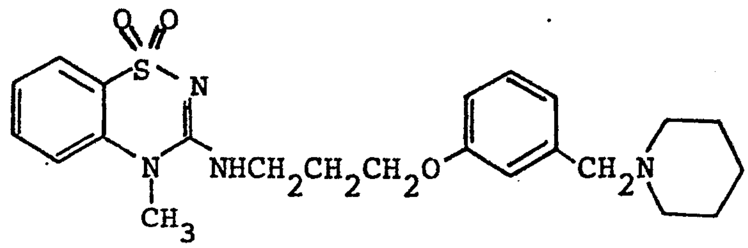

$^1H$ NMR $d^6$ DMSO

$\delta$ = 1.2-1.75 (m, 6H)
$\delta$ = 1.8-2.2 (m, 2H)
$\delta$ = 2.5-2.8 (m, 4H)
$\delta$ = 3.2-4.3 (m, 9H including 3.80, s, 3H and 4.09, t, 2H)
$\delta$ = 6.80-7.80 (m, 8H)
$\delta$ = 10.5-11.5 (m, 1H)

Mass spectrum $C_{23}H_{30}N_4O_3S$ Required 442.2039
Observed 442.2045

Example 37d

N-[3-(3-Piperid-1-ylmethylphenoxy)propyl]-7-nitro-1,2,4-benzothiadiazine-3-amine-1,1-dioxide monohydrochloride

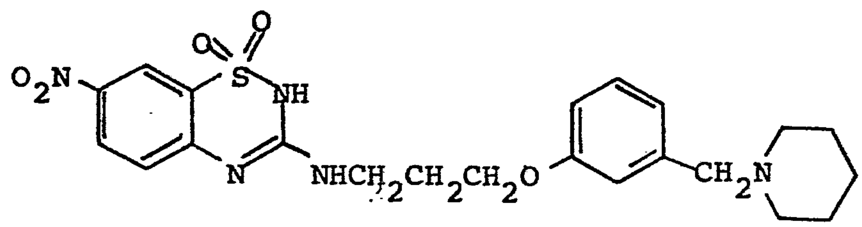

.HCl

$^1$H-NMR d$^6$ DMSO

δ = 1.25-2.3 (m, 8H including 2.05, t, 2H)
δ = 2.75-3.75 (m, 6H including 3.50, t, 2H)
δ = 4.14 (t, 2H)
δ = 4.29 (s, 2H)
δ = 6.90-7.60 (m, 5H)
δ = 7.75-8.0 (m, 1H)
δ = 8.30-8.55 (m, 2H)
δ = 10.0-12.5 (m, 2H)

Example 37e

N-[3-(3-Piperid-1-ylmethylphenoxy)propyl]-6-trifluoromethyl-1,2,4-benzothiadiazine-3-amine-1,1-dioxide monohydrochloride

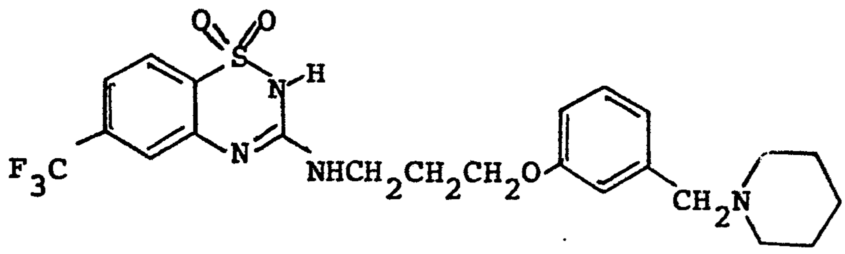

.HCl

$^1H$ NMR d$^6$DMSO

δ = 1.0-2.25 (m, 8H including 2.02, t, 2H)
δ = 2.60-3.75 (m, 6H)
δ = 3.80-4.40 (m, 4H)
δ = 6.95-8.1 (m, 8H)
δ = 10 -10.6 (m, 1H)
δ = 11.5-11.9 (m, 1H)

Mass spectrum $C_{23}H_{27}N_4O_3F_3S$ Required 496.1756
Observed 496.1760

Example 38

N-[3-(3-Piperid-1-ylmethylphenoxy)propyl]-3,7-diamino-1,2,4-benzothiadiazine-1,1-dioxide

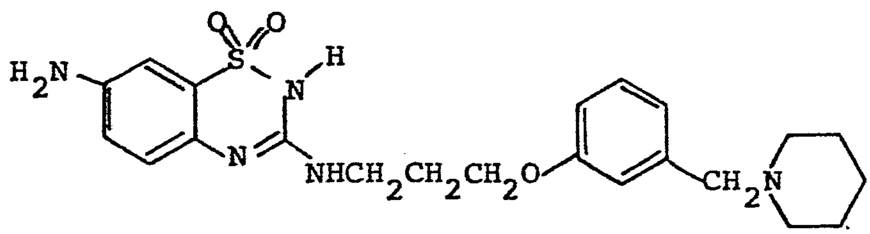

A suspension of electrolytic iron (400mgs) in a solution of N-[3-(3-piperid-1-ylmethylphenoxy)propyl]-7-nitro-1,2,4-benzothiadiazine-1,1-dioxide (0.4g) in 5N hydrochloric acid (18ml) and ethanol (15ml) was stirred at room temperature for 4 hrs. On basification ($K_2CO_3$), the product was extracted with ethylacetate (3 x 100ml) and dried ($Na_2SO_4$). Concentration and purification by column chromatography (silica, 10% $MeOH/CHCl_3$) afforded the title compound as a foam (200 mgs).

$^1H$ NMR $d^6DMSO$

$\delta$ = 1.1-1.75 (m, 6H)
$\delta$ = 1.8-2.2 (m, 2H)
$\delta$ = 2.3-2.7 (m, 4H)
$\delta$ = 3.2-3.5 (brq, 2H)
$\delta$ = 3.67 (s, 2H)
$\delta$ = 4.05 (t, 2H)
$\delta$ = 4.75-5.5 (m, 2H)
$\delta$ = 6.60-7.50 (m, 8H)
$\delta$ = 10.50 (brs, 1H)

Mass spectrum $C_{22}H_{27}N_5O_3S$ Required 443.1991
Observed 443.1988

EXAMPLE 39.

N-[2-({5-Dimethylaminomethyl furan-2-yl}methylthio)ethyl]-1,2,4-benzothiadiazine-3-amine-1,1-dioxide

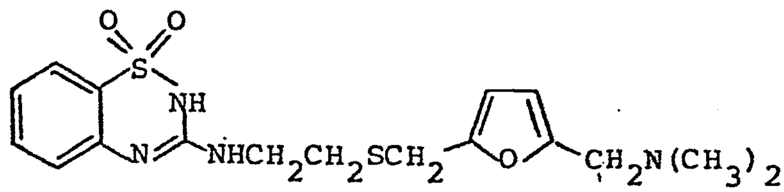

A mixture of 3-methylthio-1,2,4-benzothiadiazole-1,1-dioxide (0.25g) and 2-[(5-dimethylaminomethylfuran-2-yl) methylthio]-ethylamine (0.22g) was heated to 160° for 1 hour. On cooling, the residue was dissolved in chloroform (3ml) and purified by column chromatography (silica, chloroform/3% methanol) to give the N-[2-({5-dimethylamino-ethylfuran-2-yl}methylthio)-ethyl]-1,2,4-benzothiadiazine-3-amine-1,1-dioxide (0.20g, 55%) as a foam.

| | | |
|---|---|---|
| NMR (δ, $CDCl_3$) | 7.95-7.75 | (d,d, 1H, aromatic 8H) |
| | 7.6 -6.5 | (m, 5H, aromatic H + NH) |
| | 6.18 | (s, 2H) |
| | 3.70 | (s, 2H, N $CH_2$-aryl) |
| | 3.65-3.3 | (m, 4H, S-$CH_2$-aryl + N-$CH_2$-C≡) |
| | 2.71 | (br tr., 2H, S-$CH_2$-C≡) |
| | 2.35 | (s, 6H, $NCH_3$) |

m.s. Observed 394.1134, Theory 394.1134 for $C_{17}H_{22}N_4O_3S_2$

EXAMPLE 40

N-[3-(3-{1-piperidylmethyl}phenoxy)propyl]-1,2,4-benzothiadiazine-3-amine-1,1-dioxide

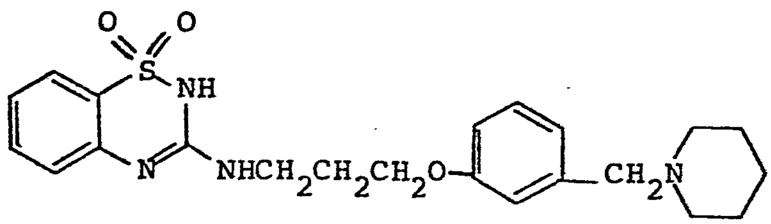

Following the procedure described in Example 39; N-[3-(3-{1-piperidylmethyl}phenoxy)propyl]-1,2,4-benzothiadiazine-3-amine-1,1-dioxide was obtained as a foam (0.2g, 47%)

| | | |
|---|---|---|
| NMR (δ, $CDCl_3$) | 7.9-6.4 | (m, 10H, aromatic $\underline{H}$ and N$\underline{H}$) |
| | 3.99 | (br tr, 2H, $OC\underline{H}_2$-C≡) |
| | 3.7-3.3 | (m, 4H, aryl-$C\underline{H}_2$-N and $NC\underline{H}_2$-C≡) |
| | 2.65-2.25 | (m, 4H, piperidyl N-$C\underline{H}_2$-C≡) |
| | 2.15-1.8 | (m, 2H, aliphatic $\underline{H}$) |
| | 1.75-1.25 | (m, 6H, aliphatic $\underline{H}$) |

Pharmacological Data Section

The ability of the compounds of the invention to modify the pH of gastric acid secretion was investigated as follows:

The perfused rat stomach preparation

The modified (1) perfused stomach preparation (2) of the urethane (25% solution) anaesthetised rat, maintained at 37°C, allows the continuous measurement of pH during basal and stimulated acid secretion.

The lumen of the stomach of male Wistar rats (approximately 200 g bodyweight) was perfused, via a cannula designed to reduce the dead space of the stomach, with 5% glucose solution (37°C) at the rate of 3 ml/min. The perfusate was forced over the surface of the secretory mucosa only, the antrum being excluded. The effluent then passed over a microflow-type glass pH eletrode via a collecting funnel situated in the non-glandular forestomach.

The secretagogue histamine was administered as a constant intravenous infusion to produce a steady rate of acid secretion. Test compounds were administered in solution as bolus intravenous injections and any effect on the pH of the perfusate noted. The perfusate pH was recorded on a potentiometric recorder and anti-secretory responses were measured in terms of the maximal reduction in hydrogen-ion concentration expressed as a percentage of the control concentration.

See Table I.

References:

1) Ghosh, M.N. and Schild, H.O. (1958). Br. J. Pharmacol., 13, 54-61.

2) Parsons, M.E. (1970).
Ph.D. Thesis, University of London.

Table I

| Compound of Example No. | Dose (μmole/kg) | Inhibition |
|---|---|---|
| 1 | 0.2 | 87% |
| 2 | 0.2 | 100% |
| 3 | 1 | 100% |
| 5 | 2 | 80% |
| 6 | 1 | 75% |
| 7 | 0.5 | 68% |
| 8 | 5 | 75% |
| 9 | 0.1 | 89% |
| 10 | 0.5 | 100% |
| 11 | 0.5 | 100% |
| 12 | 0.5 | 94% |
| 14 | 0.2 | 60% |
| 15 | 0.4 | 60% |
| 16 | 0.1 | 100% |
| 20 | 0.2 | 100% |
| 39 | 0.5 | 78% |
| 40 | 0.5 | 94% |

Guinea-pig isolated atria

This test is designed to specifically detect histamine $H_2$-receptor antagonists. Guinea-pigs of either sex, weighing between 250 and 500 g, were killed by cervical dislocation and exsanguination. The heart was removed and placed in cold McEwens solution. The

atria were dissected free and mounted in a jacketed 5 ml capacity tissue bath containing McEwens solution maintained at 32°C and gassed with a 95% oxygen, 5% carbon dioxide gas mixture. Atrial beating was detected by an auxotonic lever and from thence recorded on a Devices MX2 hot wire pen recorder. Atrial rate was derived from the amplified force signal using a Devices Instantaneous Ratemeter and recorded on the MX2 pen recorder. The addition of histamine to the tissue bath resulted in increases in both the rate and force of atrial beating. To avoid disruption of the tissue by repeated washing, responses to cumulative concentrations of histamine were obtained in the absence (control) and then presence of various concentrations of test compounds. The ability to antagonise histamine mediated positive chronotropic responses of this tissue is believed to be specific for $H_2$-receptor antagonists. Such activity was assessed by examining the ability of test compounds to shift to the right the plotted concentration/response curves to histamine. A less than 5-fold decrease in histamine potency was taken as inactive, 5-10 fold as slightly active and 10 fold or greater as active.

See Table II.

TABLE II

| Compound of Example No. | Concentration Molar | Activity (Decrease in Histamine Potency) |
|---|---|---|
| 1 | $7.5 \times 10^{-7}$ | Active |
| 2 | $7.5 \times 10^{-8}$ | Active |
| 3 | $5 \times 10^{-7}$ | Active |
| 6 | $5 \times 10^{-7}$ | Active |
| 8 | $1 \times 10^{-5}$ | Active |
| 10 | $2.5 \times 10^{-7}$ | Active |
| 11 | $1 \times 10^{-7}$ | Active |
| 12 | $1 \times 10^{-6}$ | Active |
| 13 | $1 \times 10^{-6}$ | Active |
| 14 | $5 \times 10^{-7}$ | Active |
| 16 | $7.5 \times 10^{-8}$ | Active |
| 20 | $2.5 \times 10^{-7}$ | Active |
| 39 | $1 \times 10^{-6}$ | Active |

Toxicity

No drug-induced toxic effects were observed in these tests.

## Claims

1. A compound of formula (I), or a pharmaceutically acceptable salt, quaternised derivative, N-oxide or solvate therof:

$$R_1\text{-Ar-}(CH_2)_a\text{-X-}(CH_2)_b\text{-NH-Het} \qquad (I)$$

wherein:

$R_1$ is hydrogen, halogen, hydroxy, $C_{1-4}$ alkyl, $C_{1-4}$ alkoxy, or amino or guanidino, the or any amino moiety being optionally substituted by one or two $C_{1-4}$ alkyl optionally substituted by halogen, or an aminomethylene group of formula (a),

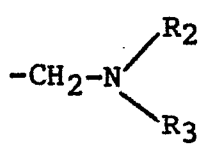

(a)

in which $R_2$ and $R_3$ are the same or different and are hydrogen, $C_{1-4}$ alkyl, $C_{3-6}$ cycloalkyl, $C_{3-6}$ cycloalkyl-$C_{1-4}$ alkyl or phenyl $C_{1-4}$ alkyl, or $R_2$ and $R_3$ together with the nitrogen atom to which they are attached are morpholino, pyrrolidino or piperidino;

Ar is furandiyl, thiophendiyl, phenylene, pyridinediyl, pyrimidinediyl, thiazolediyl, thiadiazolediyl or imidazolediyl;

a is 0 or an integer 1 or 2;

X is -S-, -O-, $-CH_2-$, $-\overset{\overset{O}{\|}}{S}-$ or -NH-;

b is an integer from 2 to 5; and

Het is a bicyclic heteroaryl group of formula (b) or (c),

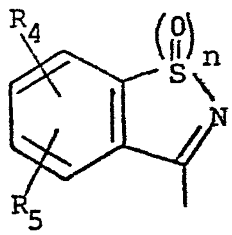

(b)

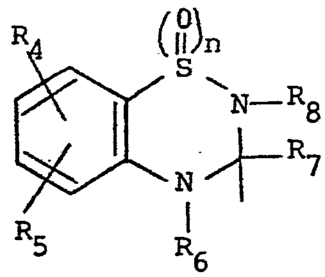

(c)

in which $R_4$ and $R_5$ are the same or different and are hydrogen, halogen, $C_{1-6}$ alkyl, trifluoromethyl, nitro, cyano, hydroxy, $C_{1-6}$ alkoxy, $C_{1-6}$ alkylthio, carboxy, amino, aminocarbonyl, aminocarbonylamino, amino-thiocarbonylamino, aminosulphonyl, $C_{1-6}$ alkylsulphonyl, $C_{1-6}$ alkylsulphinyl, $C_{1-6}$ alkylsulphonylamino, $C_{1-6}$ alkylsulphinylamino, phenylsulphonyl, phenylsulphinyl or are a group of formula (d) or (e),

$R_9$-CY- (d) $R_9$-CY-Z- (e)

in which $R_9$ is hydrogen, $C_{1-6}$ alkyl, $C_{1-6}$ alkoxy, $C_{1-6}$ alkylthio, phenyl, phenoxy or phenylthio, Y is oxygen or sulphur and Z is oxygen, sulphur or N-$R_{10}$, $R_{10}$ being hydrogen or $C_{1-6}$ alkyl, the $C_{1-6}$ alkyl group or the $C_{1-6}$ alkyl moiety of any of the foregoing $C_{1-6}$ alkyl-containing groups for $R_4$ and $R_5$ being optionally substituted by phenyl, the amino group or

the amino moiety of any of the foregoing amino-containing groups for $R_4$ and $R_5$ being optionally substituted by one or two $C_{1-6}$ alkyl, phenyl or phenyl-$C_{1-4}$ alkyl or optionally N-disubstituted by $C_{4-5}$ polymethylene, and the phenyl moiety of any of the foregoing phenyl-containing groups for $R_4$ and $R_5$ being optionally substituted by $C_{1-6}$ alkyl, $C_{1-6}$ alkoxy, halogen, nitro or trifluoromethyl, one of $R_6$ and $R_8$ is hydrogen, $C_{1-6}$ alkyl, $C_{1-6}$ alkanoyl, $C_{1-6}$ alkylsulphonyl, $C_{1-6}$ alkoxycarbonyl or phenoxycarbonyl, benzyloxycarbonyl, phenyl, phenylsulphonyl or phenyl $C_{1-4}$ alkyl, the phenyl moiety being optionally substituted by benzoyl, $C_{1-6}$ alkyl, $C_{1-4}$ alkoxy, halogen, nitro or trifluoromethyl, and the other of $R_6$ and $R_8$ together with $R_7$ is a bond, and n is 0 or an integer 1 or 2.

2. A compound according to claim 1, wherein Ar is 2,5-furandiyl; 2,5-thiophendiyl; 1,3-phenylene; or 2,4-thiazolediyl.

3. A compound according to claim 1, wherein Ar is furandiyl, thiophendiyl or phenylene and $R_1$ is an aminomethylene group of formula (a), as defined in claim 1.

4. A compound according to claim 1, wherein Ar is thiazolediyl and $R_1$ is guanidino, any amino moiety being optionally substituted by one or two $C_{1-4}$ alkyl.

5. A compound according to any one of the preceding claims, wherein a is O or the integer 1.

6. A compound according to any one of the preceding claims, wherein X is -S-, -O- or $-CH_2-$.

7. A compound according to any one of the preceding claims, wherein b is an integer 2,3 or 4.

8. A compound according to any one of the preceding claims, wherein $R_4$ and $R_5$ are the same or different and are hydrogen, trifluoromethyl, nitro, cyano, hydroxy, $C_{1-6}$ alkoxy, $C_{1-6}$ alkylthio, or amino optionally substituted by one or two $C_{1-6}$ alkyl or phenyl $C_{1-4}$ alkyl or optionally N-disubstituted by $C_{4-5}$ polymethylene, or $C_{1-6}$ alkylsulphonyl or phenylsulphonyl, the phenyl moiety being optionally substituted by $C_{1-6}$ alkyl, $C_{1-6}$ alkoxy, halogen, nitro or trifluoromethyl, or are a group of formula (d) in which Y is oxygen and $R_9$ is as defined in claim 1, or are a group of formula (e) in which Y is oxygen, Z is $N-R_{10}$ and $R_9$ and $R_{10}$ are as defined in claim 1.

9. A compound according to any one of claims 1 to 7, wherein $R_4$ and $R_5$ are the same or different and are hydrogen, halogen, $C_{1-6}$ alkyl, trifluoromethyl, nitro, cyano, $C_{1-6}$ alkoxy, or amino, aminocarbonylamino or aminosulphonyl, the amino moiety being optionally substituted by one or two $C_{1-6}$ alkyl, phenyl or phenyl-$C_{1-4}$ alkyl or optionally N-disubstituted by $C_{4-5}$ polymethylene, or $C_{1-6}$ alkylsulphonylamino or a group of formula (e) in which $R_9$ is hydrogen or $C_{1-6}$ alkyl, Y is oxygen and Z is NH.

10. A compound according to claim 9, wherein $R_4$ and $R_5$ are the same or different and are hydrogen, chloro, bromo, iodo, methyl, trifluoromethyl, nitro, cyano, methoxy, amino, methylaminocarbonylamino, aminosulphonyl, methylaminosulphonyl, dimethylaminosulphonyl, methylsulphonylamino, formamido, acetamido and propionamido.

11. A compound according to claim 10, wherein one of $R_4$ and $R_5$ is hydrogen and the other is hydrogen, nitro, amino, methylaminosulphonyl, dimethylaminosulphonyl, acetamido and propionamido.

12. A compound according to claim 1, wherein Het is a bicyclic heteroaryl group of formula (b), in which one of $R_4$ and $R_5$ is hydrogen and the other is a substituent, as defined in claim 1, in the 4- or 6-position.

13. A compound according to claim 3, wherein a is O or an integer 1, X is -S-, -O- or $-CH_2-$, b is an integer from 2 to 5 and Het is a bicyclic heteroaryl group of formula (b), in which $R_4$ and $R_5$ are the same or different and are hydrogen, trifluoromethyl, nitro, cyano, hydroxy, $C_{1-6}$ alkoxy, $C_{1-6}$ alkylthio, or amino optionally substituted by one or two $C_{1-6}$ alkyl or phenyl $C_{1-4}$ alkyl or optionally N-disubstituted by $C_{4-5}$ polymethylene, or $C_{1-6}$ alkylsulphonyl or phenylsulphonyl, the phenyl moiety being optionally substituted by $C_{1-6}$ alkyl, $C_{1-6}$ alkoxy, halogen, nitro or trifluoromethyl, or are a group of formula (d) in which Y is oxygen and $R_9$ is as defined in claim 1, or are a group of formula (e) in which Y is oxygen, Z is $N-R_{10}$ and $R_9$ and $R_{10}$ are as defined in claim 1, and n is 2.

14. A compound according to any one of the preceding claims wherein n is 1 or 2.

15. N-[2-(5-dimethylaminomethylfuran-2-ylmethylthio)-ethyl]-4-nitro-1,2-benzoisothiazole-3-amine-1,1-dioxide;

N-[3-(3-dimethylaminomethylphenoxy)propyl]-4-nitro-1,2-benzoisothiazole-3-amine-1,1-dioxide;

N-[3-(4-dimethylaminomethylphenoxy)propyl]-4-nitro-1,2-benzoisothiazole-3-amine-1,1-dioxide;

N-[3-(3-piperid-1-ylmethylphenoxy)propyl]-4-nitro-1,2-benzoisothiazole-3-amine-1,1-dioxide;

N-[2-(5-dimethylaminomethylthiophen-2-ylmethylthio)-ethyl]-4-nitro-1,2-benzoisothiazole-3-amine-1,1-dioxide;

N-[2-(5-dimethylaminomethylfuran-2-ylmethylthio)ethyl]-1,2-benzoisothiazole-3-amine-1,1-dioxide;

N-[2-(3-dimethylaminomethylphenoxy)ethyl]-4-chloro-1,2-benzoisothiazole-3-amine-1,1-dioxide;

$N^3$-[2-(5-dimethylaminomethylfuran-2-ylmethylthio)-ethyl]-1,2-benzoisothiazole-3,4-diamine-1,1-dioxide;

$N^3$-[3-(3-dimethylaminomethylphenoxy)propyl]-1,2-benzoisothiazole-3,4-diamine-1,1-dioxide;

$N^3$-[3-(4-dimethylaminomethylphenoxy)propyl]-1,2-benzoisothiazole-3,4-diamine-1,1-dioxide;

$N^3$-[3-(3-piperid-1-ylmethylphenoxy)propyl]-1,2-benzoisothiazole-3,4-diamine-1,1-dioxide;

$N^3$-[2-(5-dimethylaminomethylthiophen-2-ylmethylthio)-ethyl]-1,2-benzoisothiazole-3,4-diamine-1,1-dioxide; or

a pharmaceutically acceptable salt, quaternised derivative, N-oxide or solvate thereof.

16. N-[2-(5-dimethylaminomethylfuran-2-ylmethylthio)-ethyl]-4-chloro-1,2-benzoisothiazole-3-amine-1,1-dioxide;

N-[2-(5-dimethylaminomethylfuran-2-ylmethylthio)ethyl]-5-nitro-1,2-benzoisothiazole-3-amine-1,1-dioxide;

N-[2-(5-dimethylaminomethylfuran-2-ylmethylthio)ethyl]-6-nitro-1,2-benzoisothiazole-3-amine-1,1-dioxide;

N-[2-(5-dimethylaminomethylfuran-2-ylmethylthio)ethyl]-7-nitro-1,2-benzoisothiazole-3-amine-1,1-dioxide;

N-[2-(5-dimethylaminomethylfuran-2-ylmethylthio)ethyl]-6-chloro-1,2-benzoisothiazole-3-amine-1,1-dioxide;

N-[2-(5-dimethylaminomethylfuran-2-ylmethylthio)ethyl]-6-dimethylsulphamoyl-1,2-benzoisothiazole-3-amine-1,1-dioxide;

N-[2-(5-dimethylaminomethylfuran-2-ylmethylthio)ethyl]-4-iodo-1,2-benzoisothiazole-3-amine-1,1-dioxide;

N-[2-(5-dimethylaminomethylfuran-2-ylmethylthio)ethyl]-4-cyano-1,2-benzoisothiazole-3-amine-1,1-dioxide;

$N^3$-[2-(5-dimethylaminomethylfuran-2-ylmethylthio)-ethyl]-1,2-benzoisothiazole-3,6-diamine-1,1-dioxide;

$N^3$-[2-(5-dimethylaminomethylfuran-2-ylmethylthio)-ethyl]-1,2-benzoisothiazole-3,7-diamine-1,1-dioxide;

N-[2-(5-dimethylaminomethylfuran-2-ylmethylthio)-ethyl]-5,6-dimethoxy-1,2-benzoisothiazole-3-amine-1,1-dioxide;

$N^3$-[2-(5-dimethylaminomethylfuran-2-ylmethylthio)-ethyl]-6-acetamido-1,2-benzoisothiazole-3-amine-1,1-dioxide;

N-[2-(5-dimethylaminomethylfuran-2-ylmethylthio)-ethyl]-4-methyl-1,2-benzoisothiazole-3-amine-1,1-dioxide;

$N^3$-[2-(5-dimethylaminomethylfuran-2-ylmethylthio)-ethyl-1,2-benzoisothiazole-3,5-diamine-1,1-dioxide;

$N^3$[2-(5-dimethylaminomethylfuran-2-ylmethylthio)-ethyl]-6-methylcarbamoylamino-1,2-benzoisothiazole-3-amine-1,1-dioxide;

$N^3$-[2-(5-dimethylaminomethylfuran-2-ylmethylthio)-ethyl]-4-acetamido-1,2-benzoisothiazole-3-amine-1,1-dioxide;

N-[3-(3-piperid-1-ylmethylphenoxy)propyl]-1,2-benzoisothiazole-3-amine-1,1-dioxide;

N-[3-(3-dimethylaminomethylphenoxy)propyl]-1,2-benzoisothiazole-3-amine-1,1-dioxide;

$N^3$-[3-(3-piperid-1-ylmethylphenoxy)propyl]-1,2-benzoisothiazole-3,6-diamine-1,1-dioxide;

$N^3$[4-(3-piperid-1-ylmethylphenoxy)butyl]-1,2-benzoisothiazole-3,4-diamine-1,1-dioxide;

$N^3$-[3-(3-piperid-1-ylmethylphenoxy)propyl]-6-acetamido-1,2-benzoisothiazole-3-amine-1,1-dioxide;

$N^3$-[3-(3-piperid-1-ylmethylphenoxy)propyl]-1,2-benzoisothiazole-3-amine-1-oxide;

N-[2-(2-guanidinothiazol-4-ylmethylthio)ethyl]-4-nitro-1,2-benzoisothiazole-3-amine-1,1-dioxide;

N-[2-(2-guanidinothiazol-4-ylmethylthio)ethyl]-1,2-benzoisothiazole-3,4-diamine-1,1-dioxide; or

a pharmaceutically acceptable salt, quaternised derivative, N-oxide or solvate thereof.

17. N-[2-(5-dimethylaminomethylfuran-2-ylmethylthio)-ethyl]-6-methylsulphamoyl-1,2-benzoisothiazole-3-amine-1,1-dioxide;

$N^3$-[2-(5-dimethylaminomethylfuran-2-ylmethylthio)-ethyl]-6-methylcarbamoylamino-1,2-benzoisothiazole-3-amine-1,1-dioxide;

N-[2-(5-dimethylaminomethylfuran-2-ylmethylthio)-ethyl]-6-sulphamoyl-1,2-benzoisothiazole-3-amine-1,1-dioxide;

$N^3$-[2-(5-dimethylaminomethylfuran-2-ylmethylthio)-ethyl]-1,2-benzoisothiazole-3,4-diamine-1,1-dioxide;

N-[3-(3-piperid-1-ylmethylphenoxy)propyl]-6-dimethylsulphamoyl-1,2-benzoisothiazole-3-amine-1,1-dioxide;

N-[3-(3-piperid-1-ylmethylphenoxy)propyl]-6-methanesulphonylamino-1,2-benzoisothiazole-3-amine-1,1-dioxide;

N-[3-(3-piperid-1-ylmethylphenoxy)propyl]-6-iodo-1,2-benzoisothiazole-3-amine-1,1-dioxide;

N-[3-(3-piperid-1-ylmethylphenoxy)propyl]-6-propionamido-1,2-benzoisothiazole-3-amine-1,1-dioxide;

N-[3-(3-(1-piperidylmethyl)phenoxy)propyl]-6-N-methylsulphamoyl-1,2-benzoisothiazole-3-amine-1,1-dioxide;

N-[3-(3-(1-piperidylmethyl)phenoxy)propyl]-6-sulphamoyl-1,2-benzoisothiazole-3-amine-1,1-dioxide;

N-[2-(2-guanidinothiazole-4-ylmethylthio)ethyl]-6-dimethylsulphamoyl-1,2-benzoisothiazole-3-amine-1,1-dioxide;

N-[2-(2-guanidinothiazole-4-ylmethylthio)ethyl]-6-acetamido-1,2-benzoisothiazole-3-amine-1,1-dioxide;

N-[2-(2-guanidinothiazole-4-ylmethylthio)ethyl]-1,2-benzoisothiazole-3,6-diamine-1,1-dioxide;

N-[2-(2-guanidinothiazole-4-ylmethylthio)ethyl]-6-sulphamoyl-1,2-benzoisothiazole-3-amine-1,1-dioxide;

or a pharmaceutically acceptable salt, quaternised derivative, N-oxide or solvate thereof.

18. N-[2-((5-dimethylaminomethylfuran-2-yl)-methylthio)-ethyl)-1,2,4-benzothiadiazine-3-amine-1,1-dioxide;

N-[3-(3-(1-piperidylmethyl)phenoxy)propyl]-1,2,4-benzothiadiazine-3-amine-1,1-dioxide; or

a pharmaceuticlly acceptable salt, quaternised derivative, N-oxide or solvate thereof.

19. N-[2-(5-dimethylaminomethylfuran-2-ylmethylthio)-ethyl]-7-dimethylsulphamoyl-1,2,4-benzothiadiazine-3-amine-1,1-dioxide;

N-[3-(3-piperid-1-ylmethylphenoxy)propylamino]-7-dimethylsulphamoyl-1,2,4-benzothiadiazine-3-amine-1,1-dioxide;

N-[2-(2-guanidinothiazole-4-ylmethylthio)ethyl]-7-dimethylsulphamoyl-1,2,4-benzothiadiazine-3-amine-1,1-dioxide

N-[3-(3-piperid-1-ylmethylphenoxy)propylamino]-7-bromo-1,2,4-benzothiadiazine-3-amine-1,1-dioxide;

N-[2-((5-dimethylaminomethylfuran-2-yl)methylthio)-ethyl]-2-methyl-1,2,4-benzothiadiazine-3-amine-1,1-dioxide;

N-[2-((5-dimethylaminomethylfuran-2-yl)methylthio)-ethylamino]-4-methyl-1,2,4-benzothiadiazine-3-amine-1,1-dioxide;

N-[3-(3-piperid-1-ylmethylphenoxy)propylamino]-4-methyl-1,2,4-benzothiadiazine-3-amino-1,1-dioxide;

N-[3-(3-piperid-1-ylmethylphenoxy)propyl]-7-nitro-1,2,4-benzothiadiazine-3-amine-1,1-dioxide;

N-[3-(3-piperid-1-ylmethylphenoxy)propyl]-6-trifluoromethyl-1,2,4-benzothiadiazine-3-amine-1,1-dioxide;

N-[3-(3-piperid-1-ylmethylphenoxy)propyl]-3,7-diamino-1,2,4-benzothiadiazine-1,1-dioxide; or

a pharmaceutically acceptable salt, quaternised derivative, N-oxide or solvate thereof.

20. A process for preparing a compound of formula (I), or a pharmaceutically acceptable salt, quaternised derivative, N-oxide or solvate thereof;
(A), wherein $R_1$, Ar, a, X, b, and Het are as defined in claim 1, which comprises reacting a compound of formula (II), or an acid addition salt thereof:

$$R_1\text{-Ar-}(CH_2)_a\text{-X-}(CH_2)_b\text{-}NH_2 \qquad \text{(II)}$$

wherein $R_1$, Ar, a, X and b are as defined in claim 1, with a compound of formula (III):

$$\text{Het-}L_1 \qquad \text{(III)}$$

wherein Het is as defined in claim 1 and $L_1$ is a leaving group;

(B) wherein X is -S-, -O-, $-\overset{O}{\overset{\|}{S}}-$ or -NH-, and $R_1$, Ar, a, b and Het are as defined in claim 1, which comprises reacting a compound of formula (VII):

$$R_1\text{-Ar-}(CH_2)_a\text{-}L_2 \qquad \text{(VII)}$$

wherein $R_1$, Ar and a are as defined in claim 1 and $L_2$ is a leaving group, with a compound of formula (VIII):

$$H\text{-}X'\text{-}(CH_2)_b\text{-NH-Het} \qquad \text{(VIII)}$$

wherein b and Het are as defined in claim 1 and X' is $-S-$, $-O-$, $-\overset{O}{\overset{\|}{S}}-$ or $-NH-$;

(C) wherein $R_1$ is an aminomethylene group of formula (a), as defined in claim 1, and Ar, a, X, b and Het are as defined in claim 1, which comprises reacting a compound of formula (X):

$$L_3CH_2\text{-Ar-}(CH_2)_a\text{-X-}(CH_2)_b\text{-NH-Het} \qquad \text{(X)}$$

wherein Ar, a, X, b and Het are as defined in claim 1 and $L_3$ is a leaving group, with an amine of formula (XI):

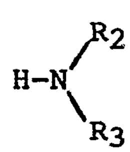

(XI)

wherein $R_2$ and $R_3$ are as defined in claim 1; or

(D) wherein $R_1$ is an aminomethylene group of formula (a), as defined in claim 1, and Ar, a, X, b and Het are as defined in claim 1, which comprises reacting a compound of formula (XV):

$$H\text{-}Ar\text{-}(CH_2)_a\text{-}X\text{-}(CH_2)_b\text{-}NH\text{-}Het \qquad (XV)$$

wherein Ar, a, X, b and Het are as defined in claim 1, with formaldehyde and a compound of formula (XI), as defined above; and

in the case where n in the resulting compound of formula (I) is 0, optionally oxidising the compound to obtain another compound of formula (I) wherein n is an integer 1 or 2; optionally converting $R_1$, $R_4$, $R_5$, $R_6$ or $R_8$ in the resulting compound of formula (I) into another $R_1$, $R_4$, $R_5$, $R_6$ or $R_8$; and optionally forming a pharmaceutically acceptable salt, quaternised derivative, N-oxide or solvate thereof.

21. A pharmaceutical composition, which comprises a compound of formula (I), as defined in any one of claims 1 to 41, or a pharmaceutically acceptable salt, quaternised derivative, N-oxide or solvate thereof, and a pharmaceutically acceptable carrier.

22. A compound of formula (I), as defined in any one of claims 1 to 19, or a pharmaceutically acceptable salt, N-oxide or solvate thereof for use in the treatment or prophylaxis of disorders in mammals such as humans caused or exacerbated by excess gastric acid secretion, such as peptic ulcer.

23. A compound of formula (III), (VIII), (X) or (XV), as defined in claim 20.